(19) **Europäisches Patentamt**
**European Patent Office**
**Office européen des brevets**

(11) **EP 3 450 451 A1**

(12) **EUROPEAN PATENT APPLICATION**
published in accordance with Art. 153(4) EPC

(43) Date of publication:
06.03.2019 Bulletin 2019/10

(21) Application number: 17789711.3

(22) Date of filing: 28.04.2017

(51) Int Cl.:
$C07K\ 14/435$ (2006.01)   $C12N\ 1/15$ (2006.01)
$C12N\ 1/19$ (2006.01)   $C12N\ 1/21$ (2006.01)
$C12N\ 5/10$ (2006.01)   $C12N\ 15/09$ (2006.01)
$D01F\ 4/02$ (2006.01)

(86) International application number:
PCT/JP2017/016917

(87) International publication number:
WO 2017/188430 (02.11.2017 Gazette 2017/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 28.04.2016 JP 2016091201
11.11.2016 JP 2016220741

(71) Applicants:
• **Spiber Inc.**
**Yamagata 997-0052 (JP)**

• **Kojima Industries Corporation**
**Toyota-shi, Aichi 471-8588 (JP)**

(72) Inventors:
• **MORITA Keisuke**
**Tsuruoka-shi**
**Yamagata 997-0052 (JP)**
• **SUGAHARA Junichi**
**Tsuruoka-shi**
**Yamagata 997-0052 (JP)**

(74) Representative: **Handley, Matthew Edward et al**
**Venner Shipley LLP**
**200 Aldersgate**
**London EC1A 4HD (GB)**

(54) **MODIFIED FIBROIN**

(57)    Provided is a modified fibroin, including a domain sequence represented by Formula 1: $[(A)_n$ motif-REP$]_m$, in which the domain sequence has an amino acid sequence having a reduced content of glycine residues equivalent to an amino acid sequence in which, at least, one or a plurality of the glycine residues in REP is substituted with another amino acid residue, as compared to naturally occurring fibroin:
[In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, and a plurality of $(A)_n$ motifs or the like may be the same amino acid sequence or different amino acid sequences.]

EP 3 450 451 A1

SEQUENCE LISTING

<110> Spiber Inc.
KOJIMA INDUSTRIES CORPORATION

<120> Altered Fibroin

<130> FP17-0267-00

<160> 11

<170> PatentIn version 3.5

<210> 1
<211> 597
<212> PRT
<213> Artificial Sequence

<220>
<223> Met-PRT313

<400> 1

```
Met Gly Pro Gly Gly Gln Gly Pro Tyr Gly Pro Gly Ala Ser Ala Ala
1               5                   10                  15


Ala Ala Ala Gly Gly Asn Gly Pro Gly Ser Gly Gln Gln Gly Pro Gly
            20                  25                  30


Gly Ser Ala Ala Ala Ala Ala Gly Gly Tyr Gly Pro Gly Gly Gln Gly
        35                  40                  45


Pro Gly Gln Gln Gly Pro Gly Ser Ser Ala Ala Ala Ala Ala Gly Pro
    50                  55                  60


Gly Gly Tyr Gly Pro Gly Gly Gln Gly Pro Ser Ala Ser Ala Ala Ala
65                  70                  75                  80


Ala Ala Gly Pro Gly Ser Gly Gln Gln Gly Pro Gly Ala Ser Ala Ala
                85                  90                  95


Ala Ala Ala Gly Gly Tyr Gly Pro Gly Gly Gln Gly Pro Gly Gln Gln
            100                 105                 110


Gly Pro Gly Ser Ser Ala Ala Ala Ala Ala Gly Gly Tyr Gly Ser Gly
        115                 120                 125


Pro Gly Gln Gln Gly Pro Tyr Gly Ser Ala Ala Ala Ala Ala Gly Pro
    130                 135                 140


Gly Ser Gly Gly Tyr Gly Gln Gly Pro Tyr Gly Pro Gly Ala Ser Ala
145                 150                 155                 160
```

```
Ala Ala Ala Ala Gly Pro Gly Gly Tyr Gly Pro Gly Gly Gln Gly Pro
                165             170                 175

Ser Ala Ser Ala Ala Ala Ala Ala Gly Ser Gly Gln Gln Gly Pro Gly
                180                 185                 190

Gly Tyr Gly Pro Tyr Ala Ser Ala Ala Ala Ala Gly Gly Tyr Gly
        195                 200                 205

Ser Gly Pro Gly Gln Gln Gly Pro Tyr Gly Pro Gly Gly Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gly Gln Gly Pro Tyr Gly
                245                 250                 255

Pro Gly Ser Ser Ala Ala Ala Ala Ala Gly Gly Tyr Gly Tyr Gly Pro
        260                 265                 270

Gly Gly Gln Gly Pro Tyr Gly Pro Gly Ala Ser Ala Ala Ala Ala Ala
        275                 280                 285

Gly Gly Asn Gly Pro Gly Ser Gly Gly Tyr Gly Pro Gly Gln Gln Gly
        290                 295                 300

Pro Gly Gly Ser Ala Ala Ala Ala Ala Gly Pro Gly Gly Gln Gly Pro
305                 310                 315                 320

Tyr Gly Pro Gly Ala Ser Ala Ala Ala Ala Gly Gly Tyr Gly Pro
        325                 330                 335

Gly Gly Gln Gly Pro Gly Gly Tyr Gly Pro Gly Ser Ser Ala Ala Ala
        340                 345                 350

Ala Ala Gly Pro Gly Gly Gln Gly Pro Tyr Gly Pro Gly Ser Ser Ala
        355                 360                 365

Ala Ala Ala Gly Gly Tyr Gly Pro Gly Gln Gln Gly Pro Tyr Gly
370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala
        405                 410                 415
```

```
Gly Pro Gly Gly Gln Gly Pro Tyr Gly Pro Gly Ala Ser Ala Ala Ala
        420                 425             430

Ala Ala Gly Pro Gly Gly Tyr Gly Pro Gly Gly Gln Gly Pro Ser Ala
        435                 440             445

Ser Ala Ala Ala Ala Ala Gly Gly Tyr Gly Ser Gly Pro Gly Gly Tyr
    450                 455             460

Gly Pro Tyr Gly Pro Gly Gly Ser Ala Ala Ala Ala Gly Pro Gly
465             470             475             480

Ser Gly Gln Gln Gly Gln Gly Pro Tyr Gly Pro Gly Ala Ser Ala Ala
            485                 490             495

Ala Ala Ala Gly Gly Tyr Gly Pro Gly Gln Gln Gly Pro Tyr Gly Pro
        500                 505             510

Gly Gly Ser Ala Ala Ala Ala Ala Gly Pro Gly Ser Gly Gly Tyr Gly
        515                 520             525

Pro Gly Ala Ser Ala Ala Ala Ala Gly Gly Asn Gly Pro Gly Ser
    530                 535             540

Gly Gly Tyr Gly Pro Gly Gln Gln Gly Pro Gly Gly Ser Ala Ala Ala
545             550                 555             560

Ala Ala Gly Gly Tyr Gln Gln Gly Pro Gly Gly Gln Gly Pro Tyr Gly
        565                 570             575

Pro Gly Ala Ser Ala Ala Ala Ala Ala Gly Pro Gly Ser Gly Gln Gln
        580                 585             590

Gly Pro Gly Ala Ser
        595
```

```
<210>  2
<211>  590
<212>  PRT
<213>  Artificial Sequence

<220>
<223>  Met-PRT399

<400>  2

Met Gly Pro Gly Gly Gln Gly Pro Tyr Gly Pro Gly Ala Ser Ala Ala
1               5               10              15
```

Ala Ala Ala Gly Gly Asn Gly Pro Gly Ser Gly Gln Gln Gly Pro Gly
                20                      25                      30

Gly Ser Gly Gly Tyr Gly Pro Gly Gly Gln Gly Pro Gly Gln Gln Gly
                35                      40                      45

Pro Gly Ser Ser Ala Ala Ala Ala Ala Gly Pro Gly Gly Tyr Gly Pro
        50                      55                      60

Gly Gly Gln Gly Pro Ser Ala Ser Ala Ala Ala Ala Ala Gly Pro Gly
65                  70                      75                      80

Ser Gly Gln Gln Gly Pro Gly Ala Ser Gly Gly Tyr Gly Pro Gly Gly
                85                      90                      95

Gln Gly Pro Gly Gln Gln Gly Pro Gly Ser Ser Ala Ala Ala Ala Ala
                100                     105                     110

Gly Gly Tyr Gly Ser Gly Pro Gly Gln Gln Gly Pro Tyr Gly Ser Ala
                115                     120                     125

Ala Ala Ala Ala Gly Pro Gly Ser Gly Gly Tyr Gly Gln Gly Pro Tyr
        130                     135                     140

Gly Pro Gly Ala Ser Gly Pro Gly Gly Tyr Gly Pro Gly Gly Gln Gly
145                     150                     155                     160

Pro Ser Ala Ser Ala Ala Ala Ala Ala Gly Ser Gly Gln Gln Gly Pro
                165                     170                     175

Gly Gly Tyr Gly Pro Tyr Ala Ser Ala Ala Ala Ala Ala Gly Gly Tyr
                180                     185                     190

Gly Ser Gly Pro Gly Gln Gln Gly Pro Tyr Gly Pro Gly Gly Ser Gly
                195                     200                     205

Ser Gly Gln Gln Gly Pro Gly Gln Gln Gly Pro Tyr Ala Ser Ala Ala
        210                     215                     220

Ala Ala Ala Gly Pro Gly Gly Gln Gly Pro Tyr Gly Pro Gly Ser Ser
225                     230                     235                     240

Ala Ala Ala Ala Ala Gly Gly Tyr Gly Tyr Gly Pro Gly Gly Gln Gly
                245                     250                     255

Pro Tyr Gly Pro Gly Ala Ser Gly Gly Asn Gly Pro Gly Ser Gly Gly
        260                     265                     270

30

Tyr Gly Pro Gly Gln Gln Gly Pro Gly Gly Ser Ala Ala Ala Ala Ala
        275             280         285

Gly Pro Gly Gly Gln Gly Pro Tyr Gly Pro Gly Ala Ser Ala Ala Ala
    290             295             300

Ala Ala Gly Gly Tyr Gly Pro Gly Gly Gln Gly Pro Gly Gly Tyr Gly
305             310             315             320

Pro Gly Ser Ser Gly Pro Gly Gly Gln Gly Pro Tyr Gly Pro Gly Ser
            325             330             335

Ser Ala Ala Ala Ala Ala Gly Gly Tyr Gly Pro Gly Gln Gln Gly Pro
        340             345             350

Tyr Gly Pro Gly Gly Ser Ala Ala Ala Ala Ala Gly Gly Tyr Gln Gln
        355             360             365

Gly Pro Gly Gly Gln Gly Pro Tyr Gly Pro Gly Ala Ser Gly Pro Gly
    370             375             380

Gly Gln Gly Pro Tyr Gly Pro Gly Ala Ser Ala Ala Ala Ala Ala Gly
385             390             395             400

Pro Gly Gly Tyr Gly Pro Gly Gly Gln Gly Pro Ser Ala Ser Ala Ala
            405             410             415

Ala Ala Ala Gly Gly Tyr Gly Ser Gly Pro Gly Gly Tyr Gly Pro Tyr
            420             425             430

Gly Pro Gly Gly Ser Gly Pro Gly Ser Gly Gln Gln Gly Gln Gly Pro
    435             440             445

Tyr Gly Pro Gly Ala Ser Ala Ala Ala Ala Gly Gly Tyr Gly Pro
    450             455             460

Gly Gln Gln Gly Pro Tyr Gly Pro Gly Gly Ser Ala Ala Ala Ala Ala
465             470             475             480

Gly Pro Gly Ser Gly Gly Tyr Gly Pro Gly Ala Ser Gly Gly Asn Gly
            485             490             495

Pro Gly Ser Gly Gly Tyr Gly Pro Gly Gln Gln Gly Pro Gly Gly Ser
        500             505             510

Ala Ala Ala Ala Ala Gly Gly Tyr Gln Gln Gly Pro Gly Gly Gln Gly
    515             520             525

Pro Tyr Gly Pro Gly Ala Ser Ala Ala Ala Ala Ala Gly Gly Tyr Gly
    530                 535                 540

Ser Gly Pro Gly Gln Gln Gly Pro Tyr Gly Pro Gly Gly Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210> 3
<211> 597
<212> PRT
<213> Artificial Sequence

<220>
<223> Met-PRT380

<400> 3

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Ala Ala Ala Ala Ala Gly Gln Tyr Gly Pro Gly Gln Gln Gly
        35                  40                  45

Pro Gly Gln Gln Gly Pro Gly Ser Ser Ala Ala Ala Ala Ala Gly Pro
    50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
            85                  90                  95

Ala Ala Ala Gly Gln Tyr Gly Pro Gly Gln Gln Gly Pro Gly Gln Gln
            100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
    130                 135                 140

```
Gly Ser Gly Gln Tyr Gly Gln Gly Pro Tyr Gly Pro Gly Ala Ser Ala
145             150             155             160

Ala Ala Ala Ala Gly Pro Gly Gln Tyr Gly Pro Gly Gln Gln Gly Pro
                165             170             175

Ser Ala Ser Ala Ala Ala Ala Ala Gly Ser Gly Gln Gln Gly Pro Gly
            180             185             190

Gln Tyr Gly Pro Tyr Ala Ser Ala Ala Ala Ala Gly Gln Tyr Gly
        195             200             205

Ser Gly Pro Gly Gln Gln Gly Pro Tyr Gly Pro Gly Gln Ser Ala Ala
    210             215             220

Ala Ala Ala Gly Ser Gly Gln Gln Gly Pro Gly Gln Gln Gly Pro Tyr
225             230             235             240

Ala Ser Ala Ala Ala Ala Ala Gly Pro Gly Gln Gln Gly Pro Tyr Gly
            245             250             255

Pro Gly Ser Ser Ala Ala Ala Ala Ala Gly Gln Tyr Gly Tyr Gly Pro
        260             265             270

Gly Gln Gln Gly Pro Tyr Gly Pro Gly Ala Ser Ala Ala Ala Ala Ala
        275             280             285

Gly Gln Asn Gly Pro Gly Ser Gly Gln Tyr Gly Pro Gly Gln Gln Gly
    290             295             300

Pro Gly Gln Ser Ala Ala Ala Ala Ala Gly Pro Gly Gln Gln Gly Pro
305             310             315             320

Tyr Gly Pro Gly Ala Ser Ala Ala Ala Ala Ala Gly Gln Tyr Gly Pro
            325             330             335

Gly Gln Gln Gly Pro Gly Gln Tyr Gly Pro Gly Ser Ser Ala Ala Ala
        340             345             350

Ala Ala Gly Pro Gly Gln Gln Gly Pro Tyr Gly Pro Gly Ser Ser Ala
        355             360             365

Ala Ala Ala Ala Gly Gln Tyr Gly Pro Gly Gln Gln Gly Pro Tyr Gly
    370             375             380

Pro Gly Gln Ser Ala Ala Ala Ala Ala Gly Gln Tyr Gln Gln Gly Pro
385             390             395             400
```

```
Gly Gln Gln Gly Pro Tyr Gly Pro Gly Ala Ser Ala Ala Ala Ala Ala
                405             410             415

Gly Pro Gly Gln Gln Gly Pro Tyr Gly Pro Gly Ala Ser Ala Ala Ala
                420             425             430

Ala Ala Gly Pro Gly Gln Tyr Gly Pro Gly Gln Gln Gly Pro Ser Ala
            435             440             445

Ser Ala Ala Ala Ala Ala Gly Gln Tyr Gly Ser Gly Pro Gly Gln Tyr
    450             455             460

Gly Pro Tyr Gly Pro Gly Gln Ser Ala Ala Ala Ala Gly Pro Gly
465             470             475             480

Ser Gly Gln Gln Gly Gln Gly Pro Tyr Gly Pro Gly Ala Ser Ala Ala
                485             490             495

Ala Ala Ala Gly Gln Tyr Gly Pro Gly Gln Gln Gly Pro Tyr Gly Pro
            500             505             510

Gly Gln Ser Ala Ala Ala Ala Ala Gly Pro Gly Ser Gly Gln Tyr Gly
    515             520             525

Pro Gly Ala Ser Ala Ala Ala Ala Gly Gln Asn Gly Pro Gly Ser
    530             535             540

Gly Gln Tyr Gly Pro Gly Gln Gln Gly Pro Gly Gln Ser Ala Ala Ala
545             550             555             560

Ala Ala Gly Gln Tyr Gln Gln Gly Pro Gly Gln Gln Gly Pro Tyr Gly
            565             570             575

Pro Gly Ala Ser Ala Ala Ala Ala Ala Gly Pro Gly Ser Gly Gln Gln
            580             585             590

Gly Pro Gly Ala Ser
        595
```

```
<210>   4
<211>   590
<212>   PRT
<213>   Artificial Sequence

<220>
<223>   Met-PRT410

<400>   4
```

```
Met Gly Pro Gly Gln Gln Gly Pro Tyr Gly Pro Gly Ala Ser Ala Ala
1                   5                   10                  15

Ala Ala Ala Gly Gln Asn Gly Pro Gly Ser Gly Gln Gln Gly Pro Gly
                20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
        50                  55                  60

Gly Gln Gln Gly Pro Ser Ala Ser Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
        130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Gly Ser Gly Gln Gln Gly Pro
            165                 170                 175

Gly Gln Tyr Gly Pro Tyr Ala Ser Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
            245                 250                 255
```

```
Pro Tyr Gly Pro Gly Ala Ser Gly Gln Asn Gly Pro Gly Ser Gly Gln
        260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
        290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
                340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
        370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
        405                 410                 415

Ala Ala Ala Gly Gln Tyr Gly Ser Gly Pro Gly Gln Tyr Gly Pro Tyr
        420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
        450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
```

36

```
                    500                     505                      510


      Ala Ala Ala Ala Ala Gly Gln Tyr Gln Gln Gly Pro Gly Gln Gln Gly
              515                 520                 525


      Pro Tyr Gly Pro Gly Ala Ser Ala Ala Ala Ala Ala Gly Gln Tyr Gly
          530                 535                 540


      Ser Gly Pro Gly Gln Gln Gly Pro Tyr Gly Pro Gly Gln Ser Gly Ser
      545                 550                 555                 560


      Gly Gln Gln Gly Pro Gly Gln Gln Gly Pro Tyr Ala Ser Ala Ala Ala
                  565                 570                 575


      Ala Ala Gly Pro Gly Ser Gly Gln Gln Gly Pro Gly Ala Ser
                  580                 585                 590


      <210>  5
      <211>  12
      <212>  PRT
      <213>  Artificial Sequence

      <220>
      <223>  HisTag

      <400>  5

      Met His His His His His His Ser Ser Gly Ser Ser
      1                   5                   10


      <210>  6
      <211>  608
      <212>  PRT
      <213>  Artificial Sequence

      <220>
      <223>  PRT313

      <400>  6

      Met His His His His His His Ser Ser Gly Ser Ser Gly Pro Gly Gly
      1                   5                   10                  15


      Gln Gly Pro Tyr Gly Pro Gly Ala Ser Ala Ala Ala Ala Ala Gly Gly
                  20                  25                  30


      Asn Gly Pro Gly Ser Gly Gln Gln Gly Pro Gly Gly Ser Ala Ala Ala
                  35                  40                  45


      Ala Ala Gly Gly Tyr Gly Pro Gly Gly Gln Gly Pro Gly Gln Gln Gly
              50                  55                  60
```

Pro Gly Ser Ser Ala Ala Ala Ala Ala Gly Pro Gly Gly Tyr Gly Pro
65              70              75              80

Gly Gly Gln Gly Pro Ser Ala Ser Ala Ala Ala Ala Ala Gly Pro Gly
            85              90              95

Ser Gly Gln Gln Gly Pro Gly Ala Ser Ala Ala Ala Ala Ala Gly Gly
            100             105             110

Tyr Gly Pro Gly Gly Gln Gly Pro Gly Gln Gln Gly Pro Gly Ser Ser
            115             120             125

Ala Ala Ala Ala Ala Gly Gly Tyr Gly Ser Gly Pro Gly Gln Gln Gly
            130             135             140

Pro Tyr Gly Ser Ala Ala Ala Ala Ala Gly Pro Gly Ser Gly Gly Tyr
145             150             155             160

Gly Gln Gly Pro Tyr Gly Pro Gly Ala Ser Ala Ala Ala Ala Ala Gly
            165             170             175

Pro Gly Gly Tyr Gly Pro Gly Gly Gln Gly Pro Ser Ala Ser Ala Ala
            180             185             190

Ala Ala Ala Gly Ser Gly Gln Gln Gly Pro Gly Gly Tyr Gly Pro Tyr
            195             200             205

Ala Ser Ala Ala Ala Ala Ala Gly Gly Tyr Gly Ser Gly Pro Gly Gln
            210             215             220

Gln Gly Pro Tyr Gly Pro Gly Gly Ser Ala Ala Ala Ala Ala Gly Ser
225             230             235             240

Gly Gln Gln Gly Pro Gly Gln Gln Gly Pro Tyr Ala Ser Ala Ala Ala
            245             250             255

Ala Ala Gly Pro Gly Gly Gln Gly Pro Tyr Gly Pro Gly Ser Ser Ala
            260             265             270

Ala Ala Ala Gly Gly Tyr Gly Tyr Gly Pro Gly Gly Gln Gly Pro
            275             280             285

Tyr Gly Pro Gly Ala Ser Ala Ala Ala Ala Gly Gly Asn Gly Pro
290             295             300

Gly Ser Gly Gly Tyr Gly Pro Gly Gln Gln Gly Pro Gly Gly Ser Ala
305             310             315             320

```
Ala Ala Ala Ala Gly Pro Gly Gly Gln Gly Pro Tyr Gly Pro Gly Ala
                325             330             335

Ser Ala Ala Ala Ala Ala Gly Gly Tyr Gly Pro Gly Gly Gln Gly Pro
                340             345             350

Gly Gly Tyr Gly Pro Gly Ser Ser Ala Ala Ala Ala Ala Gly Pro Gly
            355             360             365

Gly Gln Gly Pro Tyr Gly Pro Gly Ser Ser Ala Ala Ala Ala Ala Gly
    370             375             380

Gly Tyr Gly Pro Gly Gln Gln Gly Pro Tyr Gly Pro Gly Gly Ser Ala
385             390             395             400

Ala Ala Ala Gly Gly Tyr Gln Gln Gly Pro Gly Gly Gln Gly Pro
            405             410             415

Tyr Gly Pro Gly Ala Ser Ala Ala Ala Ala Gly Pro Gly Gly Gln
            420             425             430

Gly Pro Tyr Gly Pro Gly Ala Ser Ala Ala Ala Ala Gly Pro Gly
        435             440             445

Gly Tyr Gly Pro Gly Gly Gln Gly Pro Ser Ala Ser Ala Ala Ala Ala
    450             455             460

Ala Gly Gly Tyr Gly Ser Gly Pro Gly Gly Tyr Gly Pro Tyr Gly Pro
465             470             475             480

Gly Gly Ser Ala Ala Ala Ala Ala Gly Pro Gly Ser Gly Gln Gln Gly
            485             490             495

Gln Gly Pro Tyr Gly Pro Gly Ala Ser Ala Ala Ala Ala Gly Gly
        500             505             510

Tyr Gly Pro Gly Gln Gln Gly Pro Tyr Gly Pro Gly Gly Ser Ala Ala
    515             520             525

Ala Ala Ala Gly Pro Gly Ser Gly Gly Tyr Gly Pro Gly Ala Ser Ala
    530             535             540

Ala Ala Ala Gly Gly Asn Gly Pro Gly Ser Gly Gly Tyr Gly Pro
545             550             555             560

Gly Gln Gln Gly Pro Gly Gly Ser Ala Ala Ala Ala Ala Gly Gly Tyr
            565             570             575
```

Gln Gln Gly Pro Gly Gly Gln Gly Pro Tyr Gly Pro Gly Ala Ser Ala
        580                 585                 590

Ala Ala Ala Ala Gly Pro Gly Ser Gly Gln Gln Gly Pro Gly Ala Ser
        595                 600                 605


<210> 7
<211> 601
<212> PRT
<213> Artificial Sequence

<220>
<223> PRT399

<400> 7

Met His His His His His His Ser Ser Gly Ser Ser Gly Pro Gly Gly
1                   5                   10                  15

Gln Gly Pro Tyr Gly Pro Gly Ala Ser Ala Ala Ala Ala Gly Gly
            20                  25                  30

Asn Gly Pro Gly Ser Gly Gln Gln Gly Pro Gly Gly Ser Gly Gly Tyr
            35                  40                  45

Gly Pro Gly Gly Gln Gly Pro Gly Gln Gln Gly Pro Gly Ser Ser Ala
        50                  55                  60

Ala Ala Ala Gly Pro Gly Gly Tyr Gly Pro Gly Gly Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gly Tyr Gly Pro Gly Gly Gln Gly Pro Gly Gln
            100                 105                 110

Gln Gly Pro Gly Ser Ser Ala Ala Ala Ala Gly Gly Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gly Tyr Gly Gln Gly Pro Tyr Gly Pro Gly Ala Ser
145                 150                 155                 160

Gly Pro Gly Gly Tyr Gly Pro Gly Gly Gln Gly Pro Ser Ala Ser Ala
                165                 170                 175

```
Ala Ala Ala Ala Gly Ser Gly Gln Gln Gly Pro Gly Gly Tyr Gly Pro
            180                 185                 190

Tyr Ala Ser Ala Ala Ala Ala Ala Gly Gly Tyr Gly Ser Gly Pro Gly
            195                 200                 205

Gln Gln Gly Pro Tyr Gly Pro Gly Gly Ser Gly Ser Gly Gln Gln Gly
            210                 215                 220

Pro Gly Gln Gln Gly Pro Tyr Ala Ser Ala Ala Ala Ala Ala Gly Pro
225                 230                 235                 240

Gly Gly Gln Gly Pro Tyr Gly Pro Gly Ser Ser Ala Ala Ala Ala Ala
            245                 250                 255

Gly Gly Tyr Gly Tyr Gly Pro Gly Gly Gln Gly Pro Tyr Gly Pro Gly
            260                 265                 270

Ala Ser Gly Gly Asn Gly Pro Gly Ser Gly Gly Tyr Gly Pro Gly Gln
            275                 280                 285

Gln Gly Pro Gly Gly Ser Ala Ala Ala Ala Gly Pro Gly Gly Gln
            290                 295                 300

Gly Pro Tyr Gly Pro Gly Ala Ser Ala Ala Ala Ala Gly Gly Tyr
305                 310                 315                 320

Gly Pro Gly Gly Gln Gly Pro Gly Gly Tyr Gly Pro Gly Ser Ser Gly
            325                 330                 335

Pro Gly Gly Gln Gly Pro Tyr Gly Pro Gly Ser Ser Ala Ala Ala Ala
            340                 345                 350

Ala Gly Gly Tyr Gly Pro Gly Gln Gln Gly Pro Tyr Gly Pro Gly Gly
            355                 360                 365

Ser Ala Ala Ala Ala Ala Gly Gly Tyr Gln Gln Gly Pro Gly Gly Gln
370                 375                 380

Gly Pro Tyr Gly Pro Gly Ala Ser Gly Pro Gly Gly Gln Gly Pro Tyr
385                 390                 395                 400

Gly Pro Gly Ala Ser Ala Ala Ala Ala Ala Gly Pro Gly Gly Tyr Gly
            405                 410                 415

Pro Gly Gly Gln Gly Pro Ser Ala Ser Ala Ala Ala Ala Ala Gly Gly
            420                 425                 430
```

41

```
Tyr Gly Ser Gly Pro Gly Gly Tyr Gly Pro Tyr Gly Pro Gly Gly Ser
        435             440         445

Gly Pro Gly Ser Gly Gln Gln Gly Gln Gly Pro Tyr Gly Pro Gly Ala
    450             455             460

Ser Ala Ala Ala Ala Ala Gly Gly Tyr Gly Pro Gly Gln Gln Gly Pro
465             470             475                 480

Tyr Gly Pro Gly Gly Ser Ala Ala Ala Ala Gly Pro Gly Ser Gly
            485             490             495

Gly Tyr Gly Pro Gly Ala Ser Gly Gly Asn Gly Pro Gly Ser Gly Gly
        500             505             510

Tyr Gly Pro Gly Gln Gln Gly Pro Gly Gly Ser Ala Ala Ala Ala Ala
    515             520             525

Gly Gly Tyr Gln Gln Gly Pro Gly Gly Gln Gly Pro Tyr Gly Pro Gly
    530             535             540

Ala Ser Ala Ala Ala Ala Ala Gly Gly Tyr Gly Ser Gly Pro Gly Gln
545             550             555                 560

Gln Gly Pro Tyr Gly Pro Gly Gly Ser Gly Ser Gly Gln Gln Gly Pro
            565             570             575

Gly Gln Gln Gly Pro Tyr Ala Ser Ala Ala Ala Ala Gly Pro Gly
        580             585             590

Ser Gly Gln Gln Gly Pro Gly Ala Ser
        595             600
```

```
<210>   8
<211>   608
<212>   PRT
<213>   Artificial Sequence

<220>
<223>   PRT380

<400>   8

Met His His His His His His Ser Ser Gly Ser Ser Gly Pro Gly Gln
1               5                   10              15

Gln Gly Pro Tyr Gly Pro Gly Ala Ser Ala Ala Ala Ala Ala Gly Gln
            20              25              30
```

```
Asn Gly Pro Gly Ser Gly Gln Gln Gly Pro Gly Gln Ser Ala Ala Ala
        35                  40                  45

Ala Ala Gly Gln Tyr Gly Pro Gly Gln Gln Gly Pro Gly Gln Gln Gly
        50                  55                  60

Pro Gly Ser Ser Ala Ala Ala Ala Ala Gly Pro Gly Gln Tyr Gly Pro
65                  70                  75                  80

Gly Gln Gln Gly Pro Ser Ala Ser Ala Ala Ala Ala Gly Pro Gly
                85                  90                  95

Ser Gly Gln Gln Gly Pro Gly Ala Ser Ala Ala Ala Ala Gly Gln
                100                 105                 110

Tyr Gly Pro Gly Gln Gln Gly Pro Gly Gln Gln Gly Pro Gly Ser Ser
        115                 120                 125

Ala Ala Ala Ala Ala Gly Gln Tyr Gly Ser Gly Pro Gly Gln Gln Gly
        130                 135                 140

Pro Tyr Gly Ser Ala Ala Ala Ala Ala Gly Pro Gly Ser Gly Gln Tyr
145                 150                 155                 160

Gly Gln Gly Pro Tyr Gly Pro Gly Ala Ser Ala Ala Ala Ala Ala Gly
                165                 170                 175

Pro Gly Gln Tyr Gly Pro Gly Gln Gln Gly Pro Ser Ala Ser Ala Ala
                180                 185                 190

Ala Ala Ala Gly Ser Gly Gln Gln Gly Pro Gly Gln Tyr Gly Pro Tyr
        195                 200                 205

Ala Ser Ala Ala Ala Ala Ala Gly Gln Tyr Gly Ser Gly Pro Gly Gln
        210                 215                 220

Gln Gly Pro Tyr Gly Pro Gly Gln Ser Ala Ala Ala Ala Gly Ser
225                 230                 235                 240

Gly Gln Gln Gly Pro Gly Gln Gln Gly Pro Tyr Ala Ser Ala Ala Ala
                245                 250                 255

Ala Ala Gly Pro Gly Gln Gln Gly Pro Tyr Gly Pro Gly Ser Ser Ala
        260                 265                 270

Ala Ala Ala Gly Gln Tyr Gly Tyr Gly Pro Gly Gln Gln Gly Pro
        275                 280                 285
```

43

```
Tyr Gly Pro Gly Ala Ser Ala Ala Ala Ala Ala Gly Gln Asn Gly Pro
    290             295             300

Gly Ser Gly Gln Tyr Gly Pro Gly Gln Gln Gly Pro Gly Gln Ser Ala
305             310             315             320

Ala Ala Ala Ala Gly Pro Gly Gln Gln Gly Pro Tyr Gly Pro Gly Ala
            325             330             335

Ser Ala Ala Ala Ala Ala Gly Gln Tyr Gly Pro Gly Gln Gln Gly Pro
    340             345             350

Gly Gln Tyr Gly Pro Gly Ser Ser Ala Ala Ala Ala Ala Gly Pro Gly
    355             360             365

Gln Gln Gly Pro Tyr Gly Pro Gly Ser Ser Ala Ala Ala Ala Ala Gly
    370             375             380

Gln Tyr Gly Pro Gly Gln Gln Gly Pro Tyr Gly Pro Gly Gln Ser Ala
385             390             395             400

Ala Ala Ala Gly Gln Tyr Gln Gln Gly Pro Gly Gln Gln Gly Pro
            405             410             415

Tyr Gly Pro Gly Ala Ser Ala Ala Ala Ala Gly Pro Gly Gln Gln
    420             425             430

Gly Pro Tyr Gly Pro Gly Ala Ser Ala Ala Ala Ala Gly Pro Gly
    435             440             445

Gln Tyr Gly Pro Gly Gln Gln Gly Pro Ser Ala Ser Ala Ala Ala Ala
    450             455             460

Ala Gly Gln Tyr Gly Ser Gly Pro Gly Gln Tyr Gly Pro Tyr Gly Pro
465             470             475             480

Gly Gln Ser Ala Ala Ala Ala Ala Gly Pro Gly Ser Gly Gln Gln Gly
            485             490             495

Gln Gly Pro Tyr Gly Pro Gly Ala Ser Ala Ala Ala Ala Ala Gly Gln
            500             505             510

Tyr Gly Pro Gly Gln Gln Gly Pro Tyr Gly Pro Gly Gln Ser Ala Ala
    515             520             525

Ala Ala Ala Gly Pro Gly Ser Gly Gln Tyr Gly Pro Gly Ala Ser Ala
```

```
                    530                        535                           540


        Ala Ala Ala Ala Gly Gln Asn Gly Pro Gly Ser Gly Gln Tyr Gly Pro
        545                 550                 555                 560


        Gly Gln Gln Gly Pro Gly Gln Ser Ala Ala Ala Ala Ala Gly Gln Tyr
                        565                 570                 575


        Gln Gln Gly Pro Gly Gln Gln Gly Pro Tyr Gly Pro Gly Ala Ser Ala
                    580                 585                 590


        Ala Ala Ala Ala Gly Pro Gly Ser Gly Gln Gln Gly Pro Gly Ala Ser
                595                 600                 605


        <210>   9
        <211>   601
        <212>   PRT
        <213>   Artificial Sequence

        <220>
        <223>   PRT410

        <400>   9

        Met His His His His His His Ser Ser Gly Ser Ser Gly Pro Gly Gln
        1                   5                   10                  15


        Gln Gly Pro Tyr Gly Pro Gly Ala Ser Ala Ala Ala Ala Ala Gly Gln
                        20                  25                  30


        Asn Gly Pro Gly Ser Gly Gln Gln Gly Pro Gly Gln Ser Gly Gln Tyr
                    35                  40                  45


        Gly Pro Gly Gln Gln Gly Pro Gly Gln Gln Gly Pro Gly Ser Ser Ala
            50                  55                  60


        Ala Ala Ala Gly Pro Gly Gln Tyr Gly Pro Gly Gln Gln Gly Pro
        65                  70                  75                  80


        Ser Ala Ser Ala Ala Ala Ala Ala Gly Pro Gly Ser Gly Gln Gln Gly
                        85                  90                  95


        Pro Gly Ala Ser Gly Gln Tyr Gly Pro Gly Gln Gln Gly Pro Gly Gln
                        100                 105                 110


        Gln Gly Pro Gly Ser Ser Ala Ala Ala Ala Ala Gly Gln Tyr Gly Ser
                    115                 120                 125


        Gly Pro Gly Gln Gln Gly Pro Tyr Gly Ser Ala Ala Ala Ala Ala Gly
            130                 135                 140
```

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
            245                 250                 255

Gly Gln Tyr Gly Tyr Gly Pro Gly Gln Gln Gly Pro Tyr Gly Pro Gly
        260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
            325                 330                 335

Pro Gly Gln Gln Gly Pro Tyr Gly Pro Gly Ser Ser Ala Ala Ala Ala
        340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr

46

385     390     395     400

Gly Pro Gly Ala Ser Ala Ala Ala Ala Ala Gly Pro Gly Gln Tyr Gly
     405     410     415

Pro Gly Gln Gln Gly Pro Ser Ala Ser Ala Ala Ala Ala Ala Gly Gln
   420     425     430

Tyr Gly Ser Gly Pro Gly Gln Tyr Gly Pro Tyr Gly Pro Gly Gln Ser
   435     440     445

Gly Pro Gly Ser Gly Gln Gln Gly Gln Gly Pro Tyr Gly Pro Gly Ala
   450     455     460

Ser Ala Ala Ala Ala Ala Gly Gln Tyr Gly Pro Gly Gln Gln Gly Pro
465     470     475     480

Tyr Gly Pro Gly Gln Ser Ala Ala Ala Ala Ala Gly Pro Gly Ser Gly
     485     490     495

Gln Tyr Gly Pro Gly Ala Ser Gly Gln Asn Gly Pro Gly Ser Gly Gln
     500     505     510

Tyr Gly Pro Gly Gln Gln Gly Pro Gly Gln Ser Ala Ala Ala Ala Ala
   515     520     525

Gly Gln Tyr Gln Gln Gly Pro Gly Gln Gln Gly Pro Tyr Gly Pro Gly
   530     535     540

Ala Ser Ala Ala Ala Ala Ala Gly Gln Tyr Gly Ser Gly Pro Gly Gln
545     550     555     560

Gln Gly Pro Tyr Gly Pro Gly Gln Ser Gly Ser Gly Gln Gln Gly Pro
     565     570     575

Gly Gln Gln Gly Pro Tyr Ala Ser Ala Ala Ala Ala Ala Gly Pro Gly
     580     585     590

Ser Gly Gln Gln Gly Pro Gly Ala Ser
   595     600

<210> 10
<211> 565
<212> PRT
<213> Artificial Sequence

<220>
<223> Met-PRT468

<400> 10

| Met | Gly | Pro | Gly | Gln | Gln | Gly | Pro | Tyr | Gly | Pro | Gly | Ala | Ser | Ala | Ala |
|-----|-----|-----|-----|-----|-----|-----|-----|-----|-----|-----|-----|-----|-----|-----|-----|
| 1   |     |     |     | 5   |     |     |     |     | 10  |     |     |     |     | 15  |     |

| Ala | Ala | Ala | Ala | Ala | Gly | Ser | Asn | Gly | Pro | Gly | Ser | Gly | Gln | Gln | Gly |
|-----|-----|-----|-----|-----|-----|-----|-----|-----|-----|-----|-----|-----|-----|-----|-----|
|     |     |     | 20  |     |     |     |     | 25  |     |     |     |     | 30  |     |     |

| Pro | Gly | Gln | Ser | Gly | Gln | Tyr | Gly | Pro | Gly | Gln | Gln | Gly | Pro | Gly | Gln |
|-----|-----|-----|-----|-----|-----|-----|-----|-----|-----|-----|-----|-----|-----|-----|-----|
|     |     | 35  |     |     |     |     | 40  |     |     |     |     | 45  |     |     |     |

| Gln | Gly | Pro | Gly | Ser | Ser | Ala | Ala | Ala | Ala | Ala | Ala | Gly | Pro | Gly |
|-----|-----|-----|-----|-----|-----|-----|-----|-----|-----|-----|-----|-----|-----|-----|
|     | 50  |     |     |     |     | 55  |     |     |     |     | 60  |     |     |     |

| Gln | Tyr | Gly | Pro | Gly | Gln | Gln | Gly | Pro | Ser | Ala | Ser | Ala | Ala | Ala | Ala |
|-----|-----|-----|-----|-----|-----|-----|-----|-----|-----|-----|-----|-----|-----|-----|-----|
| 65  |     |     |     |     | 70  |     |     |     | 75  |     |     |     |     | 80  |     |

| Ala | Ala | Ala | Gly | Pro | Gly | Ser | Gly | Gln | Gln | Gly | Pro | Gly | Ala | Ser | Gly |
|-----|-----|-----|-----|-----|-----|-----|-----|-----|-----|-----|-----|-----|-----|-----|-----|
|     |     |     |     | 85  |     |     |     |     | 90  |     |     |     |     | 95  |     |

| Gln | Tyr | Gly | Pro | Gly | Gln | Gln | Gly | Pro | Gly | Gln | Gln | Gly | Pro | Gly | Ser |
|-----|-----|-----|-----|-----|-----|-----|-----|-----|-----|-----|-----|-----|-----|-----|-----|
|     |     |     | 100 |     |     |     |     | 105 |     |     |     |     | 110 |     |     |

| Ser | Ala | Ala | Ala | Ala | Ala | Ala | Ala | Gly | Ser | Tyr | Gly | Ser | Gly | Pro | Gly |
|-----|-----|-----|-----|-----|-----|-----|-----|-----|-----|-----|-----|-----|-----|-----|-----|
|     |     | 115 |     |     |     |     | 120 |     |     |     |     | 125 |     |     |     |

| Gln | Gln | Gly | Pro | Tyr | Gly | Ser | Ala | Ala | Ala | Ala | Ala | Ala | Gly | Pro |
|-----|-----|-----|-----|-----|-----|-----|-----|-----|-----|-----|-----|-----|-----|-----|
|     | 130 |     |     |     |     | 135 |     |     |     |     | 140 |     |     |     |

| Gly | Ser | Gly | Gln | Tyr | Gly | Gln | Gly | Pro | Tyr | Gly | Pro | Gly | Ala | Ser | Gly |
|-----|-----|-----|-----|-----|-----|-----|-----|-----|-----|-----|-----|-----|-----|-----|-----|
| 145 |     |     |     |     | 150 |     |     |     |     | 155 |     |     |     |     | 160 |

| Pro | Gly | Gln | Tyr | Gly | Pro | Gly | Gln | Gln | Gly | Pro | Ser | Ala | Ser | Ala | Ala |
|-----|-----|-----|-----|-----|-----|-----|-----|-----|-----|-----|-----|-----|-----|-----|-----|
|     |     |     |     | 165 |     |     |     |     | 170 |     |     |     |     | 175 |     |

| Ala | Ala | Ala | Ala | Ala | Gly | Ser | Gly | Gln | Gln | Gly | Pro | Gly | Gln | Tyr | Gly |
|-----|-----|-----|-----|-----|-----|-----|-----|-----|-----|-----|-----|-----|-----|-----|-----|
|     |     |     | 180 |     |     |     |     | 185 |     |     |     |     | 190 |     |     |

| Pro | Tyr | Ala | Ser | Ala | Ala | Ala | Ala | Ala | Ala | Ala | Gly | Ser | Tyr | Gly | Ser |
|-----|-----|-----|-----|-----|-----|-----|-----|-----|-----|-----|-----|-----|-----|-----|-----|
|     |     | 195 |     |     |     |     | 200 |     |     |     |     | 205 |     |     |     |

| Gly | Pro | Gly | Gln | Gln | Gly | Pro | Tyr | Gly | Pro | Gly | Gln | Ser | Gly | Ser | Gly |
|-----|-----|-----|-----|-----|-----|-----|-----|-----|-----|-----|-----|-----|-----|-----|-----|
|     | 210 |     |     |     |     | 215 |     |     |     |     | 220 |     |     |     |     |

| Gln | Gln | Gly | Pro | Gly | Gln | Gln | Gly | Pro | Tyr | Ala | Ser | Ala | Ala | Ala | Ala |
|-----|-----|-----|-----|-----|-----|-----|-----|-----|-----|-----|-----|-----|-----|-----|-----|
| 225 |     |     |     |     | 230 |     |     |     |     | 235 |     |     |     |     | 240 |

| Ala | Ala | Ala | Gly | Pro | Gly | Gln | Gln | Gly | Pro | Tyr | Gly | Pro | Gly | Ser | Ser |
|-----|-----|-----|-----|-----|-----|-----|-----|-----|-----|-----|-----|-----|-----|-----|-----|

                    245                          250                          255

Ala Ala Ala Ala Ala Ala Ala Gly Ser Tyr Gly Tyr Gly Pro Gly Gln
            260                  265                  270

Gln Gly Pro Tyr Gly Pro Gly Ala Ser Gly Gln Asn Gly Pro Gly Ser
            275                  280                  285

Gly Gln Tyr Gly Pro Gly Gln Gln Gly Pro Gly Pro Ser Ala Ala Ala
        290                  295                  300

Ala Ala Ala Ala Gly Pro Gly Gln Gln Gly Pro Tyr Gly Pro Gly Ala
305                  310                  315                  320

Ser Ala Ala Ala Ala Ala Ala Ala Gly Ser Tyr Gly Pro Gly Gln Gln
                325                  330                  335

Gly Pro Gly Gln Tyr Gly Pro Gly Ser Ser Gly Pro Gly Gln Gln Gly
            340                  345                  350

Pro Tyr Gly Pro Gly Ser Ser Ala Ala Ala Ala Ala Ala Ala Gly Ser
        355                  360                  365

Tyr Gly Pro Gly Gln Gln Gly Pro Tyr Gly Pro Gly Pro Ser Ala Ala
    370                  375                  380

Ala Ala Ala Ala Ala Gly Ser Tyr Gln Gln Gly Pro Gly Gln Gln Gly
385                  390                  395                  400

Pro Tyr Gly Pro Gly Ala Ser Gly Pro Gly Gln Gln Gly Pro Tyr Gly
        405                  410                  415

Pro Gly Ala Ser Ala Ala Ala Ala Ala Ala Ala Gly Pro Gly Gln Tyr
            420                  425                  430

Gly Pro Gly Gln Gln Gly Pro Ser Ala Ser Ala Ala Ala Ala Ala Ala
        435                  440                  445

Ala Gly Ser Tyr Gly Ser Gly Pro Gly Gln Tyr Gly Pro Tyr Gly Pro
    450                  455                  460

Gly Gln Ser Gly Pro Gly Ser Gly Gln Gln Gly Gln Gly Pro Tyr Gly
465                  470                  475                  480

Pro Gly Ala Ser Ala Ala Ala Ala Ala Ala Ala Gly Ser Tyr Gly Pro
            485                  490                  495

Gly Gln Gln Gly Pro Tyr Gly Pro Gly Pro Ser Ala Ala Ala Ala Ala
         500             505             510

Ala Ala Gly Pro Gly Ser Gly Gln Tyr Gly Pro Gly Ala Ser Gly Gln
         515             520             525

Asn Gly Pro Gly Ser Gly Gln Tyr Gly Pro Gly Gln Gln Gly Pro Gly
         530             535             540

Pro Ser Ala Ala Ala Ala Ala Ala Gly Pro Gly Ser Gly Gln Gln
545             550             555             560

Gly Pro Gly Ala Ser
                565


<210> 11
<211> 576
<212> PRT
<213> Artificial Sequence

<220>
<223> PRT468

<400> 11

Met His His His His His His Ser Ser Gly Ser Ser Gly Pro Gly Gln
1               5                   10              15

Gln Gly Pro Tyr Gly Pro Gly Ala Ser Ala Ala Ala Ala Ala Ala Ala
             20              25              30

Gly Ser Asn Gly Pro Gly Ser Gly Gln Gln Gly Pro Gly Gln Ser Gly
         35              40              45

Gln Tyr Gly Pro Gly Gln Gln Gly Pro Gly Gln Gln Gly Pro Gly Ser
         50              55              60

Ser Ala Ala Ala Ala Ala Ala Gly Pro Gly Gln Tyr Gly Pro Gly
65              70              75              80

Gln Gln Gly Pro Ser Ala Ser Ala Ala Ala Ala Ala Ala Gly Pro
             85              90              95

Gly Ser Gly Gln Gln Gly Pro Gly Ala Ser Gly Gln Tyr Gly Pro Gly
             100             105             110

Gln Gln Gly Pro Gly Gln Gln Gly Pro Gly Ser Ser Ala Ala Ala Ala
         115             120             125

Ala Ala Ala Gly Ser Tyr Gly Ser Gly Pro Gly Gln Gln Gly Pro Tyr

```
                130                      135                      140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala
            180                 185                 190

Gly Ser Gly Gln Gln Gly Pro Gly Gln Tyr Gly Pro Tyr Ala Ser Ala
            195                 200                 205

Ala Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Gly Pro
            245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Gly
305                 310                 315                 320

Pro Gly Gln Gln Gly Pro Tyr Gly Pro Gly Ala Ser Ala Ala Ala Ala
            325                 330                 335

Ala Ala Ala Gly Ser Tyr Gly Pro Gly Gln Gln Gly Pro Gly Gln Tyr
        340                 345                 350

Gly Pro Gly Ser Ser Gly Pro Gly Gln Gln Gly Pro Tyr Gly Pro Gly
        355                 360                 365

Ser Ser Ala Ala Ala Ala Ala Ala Ala Gly Ser Tyr Gly Pro Gly Gln
    370                 375                 380
```

```
Gln Gly Pro Tyr Gly Pro Gly Pro Ser Ala Ala Ala Ala Ala Ala
385             390         395                     400

Gly Ser Tyr Gln Gln Gly Pro Gly Gln Gln Gly Pro Tyr Gly Pro Gly
            405             410             415

Ala Ser Gly Pro Gly Gln Gln Gly Pro Tyr Gly Pro Gly Ala Ser Ala
            420             425             430

Ala Ala Ala Ala Ala Ala Gly Pro Gly Gln Tyr Gly Pro Gly Gln Gln
            435             440             445

Gly Pro Ser Ala Ser Ala Ala Ala Ala Ala Ala Ala Gly Ser Tyr Gly
    450             455             460

Ser Gly Pro Gly Gln Tyr Gly Pro Tyr Gly Pro Gly Gln Ser Gly Pro
465             470             475             480

Gly Ser Gly Gln Gln Gly Gln Gly Pro Tyr Gly Pro Gly Ala Ser Ala
            485             490             495

Ala Ala Ala Ala Ala Ala Gly Ser Tyr Gly Pro Gly Gln Gln Gly Pro
            500             505             510

Tyr Gly Pro Gly Pro Ser Ala Ala Ala Ala Ala Ala Ala Gly Pro Gly
    515             520             525

Ser Gly Gln Tyr Gly Pro Gly Ala Ser Gly Gln Asn Gly Pro Gly Ser
    530             535             540

Gly Gln Tyr Gly Pro Gly Gln Gln Gly Pro Gly Pro Ser Ala Ala Ala
545             550             555             560

Ala Ala Ala Ala Gly Pro Gly Ser Gly Gln Gln Gly Pro Gly Ala Ser
            565             570             575
```

## Description

### Technical Field

The present invention relates to a modified fibroin. More specifically, the present invention relates to a modified fibroin having a reduced content of glycine residues. The present invention also relates to a nucleic acid encoding a modified fibroin, an expression vector including the nucleic acid sequence, a host transformed with the expression vector, and a product made from a modified fibroin.

### Background Art

Fibroin is a type of fibrous protein and contains up to 90% of glycine, alanine and serine residues leading to the formation of a β-pleated sheet (Non-Patent Literature 1). Proteins (silk proteins, Hornet silk proteins, and spider silk proteins) and the like constituting the yarn produced by insects and spiders are known as fibroin.

Silk proteins have excellent mechanical properties, hygroscopic properties and deodorizing properties and are widely used as raw materials for garments. In addition, the silk yarn is an immuno-tolerant natural fiber and has high biocompatibility and is therefore also used for surgical sutures.

Up to seven types of silk glands exist in spider, each producing fibroin (spider silk protein) with different properties. According to the organ of the source, spider silk proteins are designated a major ampullate spider protein (MaSp) with high toughness, a minor ampullate spider protein (MiSp) with high elongation, and flagelliform (Flag), tubuliform, aggregate, aciniform, and pyriform spider silk proteins. In particular, structural studies have been intensively conducted in the major ampullate spider protein exhibiting high toughness due to having excellent strength (stress and toughness) and elongation (Patent Literature 1 and 2).

As a structure specific to fibroin, a structure in which amino acid motifs classified as GPGXX, an extended region rich in alanine residues (($A)_n$ or ($GA)_n$), GGX, and a spacer are repeated is known (Non-Patent Literature 2). In addition, it has been reported that substitution of the ($GA)_n$ motif with the ($A)_n$ motif leads to decreased elongation but increased tensile strength, an increasing number of GPGXX motifs leads to increased elongation, and substitution of several GPGXX motifs with the ($A)_n$ motifs leads to increased tensile strength (Patent Literature 2). In addition, the GGX and GPGXX motifs are thought to have a flexible helical structure that imparts elasticity to yarns (Patent Literature 3).

Recombinant spider silk proteins and recombinant silk proteins are produced in several heterologous protein production systems. For example, transgenic goat, transgenic silkworm, or recombinant plant or mammalian cells are utilized (Non-Patent Literature 3). However, these production systems exhibit a low production rate and are not suitable for mass production meeting the commercial level (Patent Literature 4 and Patent Literature 5). Although many cases of production of recombinant fibroin by organisms such as yeast, mold, gram-negative bacterium and gram-positive bacterium as a production system capable of mass production have also been reported and certain outcomes have been achieved, it has not been possible to achieve industrial mass production of the recombinant fibroin having excellent elongation and tensile strength (Patent Literature 5).

### Citation List

### Patent Literature

[Patent Literature 1] Japanese Unexamined Patent Publication No. 2012-55269

[Patent Literature 2] Japanese Unexamined Patent Publication No. 2005-502347

[Patent Literature 3] Japanese Unexamined Patent Publication No. 2009-505668

[Patent Literature 4] Japanese Unexamined Patent Publication No. 2014-502140

[Patent Literature 5] International Patent Publication No. WO2015/042164

### Non Patent Literature

[Non-Patent Literature 1] Asakura et al., Encyclopedia of Agricultural Science, Academic Press: New York, NY, 1994, Vol. 4, pp. 1-11

[Non-Patent Literature 2] Microbial Cell Factories, 2004, 3:14

[Non-Patent Literature 3] Science, 2002, Vol. 295, pp. 472-476

**Summary of Invention**

**Problems to be Solved by the Invention**

Due to its excellent properties, fibroin has drawn attention as a new material in various industrial fields such as medicine, aviation, and clothing. However, it is necessary to further improve the productivity of fibroin in order to achieve an amount of production that meets the commercial level.

An object of the present invention is to provide a modified fibroin having improved productivity while maintaining the strength and elongation of fibroin.

**Means for Solving the Problems**

As a result of various studies on methods capable of industrial mass production, the present inventors have unexpectedly found that the productivity of fibroin can be improved while maintaining strength (stress and toughness) and elongation by modifying the GGX motif or GPGXX motif, which is considered to be involved in elongation of fibroin, to reduce the content of glycine residues. The present invention is based on such novel findings.

That is, the present invention relates to, for example, each of the following inventions.

[1] A modified fibroin, including:

a domain sequence represented by Formula 1: $[(A)_n \text{ motif-REP}]_m$,
in which the domain sequence has an amino acid sequence having a reduced content of glycine residues equivalent to an amino acid sequence in which, at least, one or a plurality of the glycine residues in REP is substituted with another amino acid residue, as compared to naturally occurring fibroin.

[In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

[2] The modified fibroin according to [1], in which the domain sequence has an amino acid sequence equivalent to an amino acid sequence in which, at least, in at least one motif sequence selected from GGX and GPGXX (where X represents an amino acid residue other than glycine) in REP, one glycine residue in one or a plurality of the motif sequences is substituted with another amino acid residue, as compared to the naturally occurring fibroin.

[3] The modified fibroin according to [2], in which the ratio of the motif sequence having the substitution of a glycine residue with another amino acid residue is 10% or more with respect to the entire motif sequence.

[4] A modified fibroin, including:

a domain sequence represented by Formula 1: $[(A)_n \text{ motif-REP}]_m$,
in which z/w is 50.9% or more in the case where the total number of amino acid residues in the amino acid sequence consisting of XGX (where X represents an amino acid residue other than glycine) contained in all REPs in the sequence excluding the sequence from the $(A)_n$ motif located at the most C-terminal side to the C-terminal of the domain sequence from the domain sequence is defined as z, and the total number of amino acid residues in the sequence excluding the sequence from the $(A)_n$ motif located at the most C-terminal side to the C-terminal of the domain sequence from the domain sequence is defined as w.

[In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

[5] The modified fibroin according to any one of [1] to [4], in which the fibroin has, in addition to an amino acid sequence corresponding to substitution of one or a plurality of glycine residues in REP with another amino acid residue, an amino acid sequence corresponding to substitution, deletion, insertion and/or addition of one or a plurality of amino acid residues, as compared to naturally occurring fibroin.

[6] The modified fibroin according to [5], in which the naturally occurring fibroin is a fibroin derived from an insect or a spider.

[7] The modified fibroin according to [5], in which the naturally occurring fibroin is a major ampullate spider protein (MaSp) or minor ampullate spider protein (MiSp) of spiders.

[8] The modified fibroin according to any one of [5] to [7], in which the domain sequence has an amino acid sequence equivalent to an amino acid sequence in which, at least, in at least one motif sequence selected from GGX and GPGXX (where X represents an amino acid residue other than glycine) in REP, one glycine residue in one or a plurality of the motif sequences is substituted with another amino acid residue, as compared to the naturally occurring fibroin, and in which the ratio of the motif sequence having the substitution of a glycine residue with another amino acid residue is 10% or more with respect to the entire motif sequence.

[9] The modified fibroin according to [8], in which the another amino acid residue is an amino acid residue selected from the group consisting of a glutamine (Q) residue, a valine (V) residue, a leucine (L) residue, an isoleucine (I) residue, a methionine (M) residue, a proline (P) residue, a phenylalanine (F) residue, a tryptophan (W) residue, an asparagine (N) residue, a serine (S) residue, a lysine (K) residue and a glutamic acid (E) residue.

[10] The modified fibroin according to [8], in which the another amino acid residue is a glutamine (Q) residue.

[11] The modified fibroin according to any one of [1] to [10], in which the domain sequence further has an amino acid sequence having a reduced content of $(A)_n$ motif equivalent to an amino acid sequence in which, at least, one or a plurality of the $(A)_n$ motifs is deleted, as compared to the naturally occurring fibroin.

[12] The modified fibroin according to [11], in which the domain sequence has an amino acid sequence equivalent to an amino acid sequence in which, at least, one $(A)_n$ motif per one to three $(A)_n$ motifs from an N-terminal side to the C-terminal side is deleted, as compared to the naturally occurring fibroin.

[13] The modified fibroin according to [11], in which the domain sequence has an amino acid sequence equivalent to an amino acid sequence in which, at least, two consecutive $(A)_n$ motif deletions and one $(A)_n$ motif deletion are repeated in this order from an N-terminal side to the C-terminal side, as compared to the naturally occurring fibroin.

[14] The modified fibroin according to any one of [11] to [13], in which a maximum value of x/y is 20% or more, in the case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the total value of the number of amino acid residues in the two adjacent $[(A)_n$ motif-REP] units where the ratio of the number of amino acid residues in the other REP is 1.8 to 11.3 is defined as x, and the total number of amino acid residues of the domain sequence is defined as y.

[15] A modified fibroin, including an amino acid sequence set forth in SEQ ID NO: 3, SEQ ID NO: 4 or SEQ ID NO: 10, or an amino acid sequence having 90% or more sequence identity with the amino acid sequence set forth in SEQ ID NO: 3, SEQ ID NO: 4 or SEQ ID NO: 10.

[16] The modified fibroin according to any one of [1] to [15], further including a tag sequence at either or both of the N-terminal and the C-terminal.

[17] The modified fibroin according to [16], in which the tag sequence includes an amino acid sequence set forth in SEQ ID NO: 5.

[18] A modified fibroin, including an amino acid sequence set forth in SEQ ID NO: 8, SEQ ID NO: 9 or SEQ ID NO: 11, or an amino acid sequence having 90% or more sequence identity with the amino acid sequence set forth in SEQ ID NO: 8, SEQ ID NO: 9 or SEQ ID NO: 11.

[19] A nucleic acid encoding the modified fibroin according to any one of [1] to [18].

[20] A nucleic acid that hybridizes with a complementary strand of the nucleic acid according to [19] under stringent conditions and encodes a modified fibroin including a domain sequence represented by Formula 1: $[(A)_n$ motif-REP]$_m$. [In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

[21] A nucleic acid having 90% or more sequence identity with the nucleic acid according to [19] and encoding a modified fibroin including a domain sequence represented by Formula 1: $[(A)_n$ motif-REP]$_m$.

[In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

[22] An expression vector, including the nucleic acid sequence according to any one of [19] to [21] and one or a plurality of regulatory sequences operably linked thereto.

[23] The expression vector according to [22], which is a plasmid vector or a viral vector.

[24] A host transformed with the expression vector according to [22] or [23].

[25] The host according to [24], which is a prokaryote.

[26] The host according to [25], in which the prokaryote is a microorganism belonging to a genus selected from the group consisting of *Escherichia, Brevibacillus, Serratia, Bacillus, Microbacterium, Brevibacterium, Corynebacterium* and *Pseudomonas.*

[27] The host according to [24], which is a eukaryote.

[28] The host according to [27], in which the eukaryote is a yeast, a filamentous fungus or an insect cell.

[29] The host according to [28], in which the yeast is a yeast belonging to a genus selected from the group consisting of *Saccharomyces, Schizosaccharomyces, Kluyveromyces, Trichosporon, Schwanniomyces, Pichia, Candida, Yarrowia* and *Hansenula.*

[30] The host according to [29], in which the yeast belonging to the genus *Saccharomyces* is *Saccharomyces cerevisiae,* the yeast belonging to the genus *Schizosaccharomyces* is *Schizosaccharomyces pombe,* and the yeast belonging to the genus *Kluyveromyces* is *Kluyveromyces lactis*, the yeast belonging to the genus *Trichosporon* is *Trichosporon pullulans,* the yeast belonging to the genus *Schwaniomyces* is *Schwanniomyces alluvius,* the yeast belonging to the genus *Pichia* is *Pichia pastoris,* the yeast belonging to the genus *Candida* is *Candida albicans,* the yeast belonging to the genus *Yarrowia* is *Yarrowia lipolytica,* and the yeast belonging to the genus *Hansenula* is *Hansenula polymorpha.*

[31] The host according to [28], in which the filamentous fungus is a filamentous fungus belonging to a genus selected from the group consisting of *Aspergillus, Penicillium* and *Mucor.*

[32] The host according to [31], in which the filamentous fungus belonging to the genus *Aspergillus* is *Aspergillus oryzae,* the filamentous fungus belonging to the genus *Penicillium* is *Penicillium chrysogenum,* and the filamentous fungus belonging to the genus *Mucor* is *Mucor fragilis.*

[33] The host according to [28], in which the insect cell is a lepidopteran insect cell.

[34] The host according to [28], in which the insect cell is an insect cell derived from *Spodoptera frugiperda* or an insect cell derived from *Trichoplusia ni.*

[35] A product including the modified fibroin according to any one of [1] to [18] and selected from the group consisting of a fiber, a yarn, a filament, a film, a foam, a sphere, a nanofibril, a hydrogel, a resin and an equivalent thereof.

## Effects of the Invention

According to the present invention, it is possible to provide a modified fibroin having improved productivity while maintaining the strength and elongation of fibroin. Since the GGX motif and the GPGXX motif of fibroin have been considered to be involved in the elongation of the fibroin fiber, substitution of the glycine residue (G) of these motifs with another amino acid residue has been thought to greatly affect the elongation of this fibroin fiber. However, the present inventors have found that substitution of one G in the GGX motif and GPGXX motif with another amino acid does not affect the elongation of the fibroin fiber by leaving the other G remaining, and additionally the amount of production in the recombinant protein production system can be significantly improved. According to the present invention, such an unexpected effect is exerted.

## Brief Description of Drawings

FIG. 1 is a diagram showing a distribution of values of z/w (%) of naturally occurring fibroin.
FIG. 2 is a schematic diagram showing a domain sequence of a modified fibroin.
FIG. 3 is a diagram showing a distribution of values of x/y (%) of naturally occurring fibroin.

## Embodiments for Carrying Out the Invention

Hereinafter, embodiments for carrying out the present invention will be described in detail. However, the present invention is not limited to the following embodiments.

[Modified fibroin]

The modified fibroin according to the present invention is a protein including a domain sequence represented by Formula 1: $[(A)_n$ motif-REP$]_m$. In the modified fibroin, an amino acid sequence (N-terminal sequence and C-terminal sequence) may be further added to either or both of the N-terminal side and the C-terminal side of the domain sequence. The N-terminal sequence and the C-terminal sequence, although not limited thereto, are typically regions that do not have repetitions of amino acid motifs characteristic of fibroin and consist of amino acids of about 100 residues.

The term "modified fibroin" as used herein means a fibroin whose domain sequence is different from the amino acid sequence of naturally occurring fibroin. The term "naturally occurring fibroin" as used herein is also a protein including a domain sequence represented by Formula 1: $[(A)_n$ motif-REP$]_m$.

The "modified fibroin" may be a fibroin whose amino acid sequence has been modified based on naturally occurring fibroin (for example, a fibroin whose amino acid sequence has been modified by altering a gene sequence of cloned naturally occurring fibroin) or a fibroin artificially designed and synthesized independently of naturally occurring fibroin (for example, a fibroin having a desired amino acid sequence by chemically synthesizing a nucleic acid encoding the designed amino acid sequence), as long as it has the amino acid sequence specified in the present invention.

The term "domain sequence" as used herein refers to an amino acid sequence which produces a crystalline region (which typically corresponds to $(A)_n$ motif of an amino acid sequence) and an amorphous region (which typically corresponds to REP of an amino acid sequence) peculiar to fibroin and means an amino acid sequence represented by Formula 1: $[(A)_n$ motif-REP$]_m$. Here, the $(A)_n$ motif represents an amino acid sequence consisting of 4 to 20 amino acid residues and the number of alanine residues relative to the total number of amino acid residues in the $(A)_n$ motif is 83% or more. The REP represents an amino acid sequence consisting of 10 to 200 amino acid residues. m represents an integer of 8 to 300. A plurality of $(A)_n$ motifs may be the same amino acid sequence or different amino acid sequences. A plurality of REPs may be the same amino acid sequence or different amino acid sequences.

The $(A)_n$ motif may be such that the number of alanine residues is 83% or more relative to the total number of amino acid residues in the $(A)_n$ motif, preferably 86% or more, more preferably 90% or more, still more preferably 95% or more, and even still more preferably 100% (which means that the $(A)_n$ motif consists of only alanine residues). It is preferred that at least seven of a plurality of $(A)_n$ motifs in the domain sequence consist of only alanine residues. The phrase "consist of only alanine residues" means that the $(A)_n$ motif has an amino acid sequence represented by $(A)_n$ (where A represents an alanine residue and n represents an integer of 4 to 20 and preferably an integer of 4 to 16).

The modified fibroin according to one embodiment has an amino acid sequence whose domain sequence has a reduced content of glycine residues as compared to naturally occurring fibroin. The modified fibroin can be said to have an amino acid sequence equivalent to an amino acid sequence in which, at least, one or a plurality of glycine residues in REP is substituted with another amino acid residue, as compared to naturally occurring fibroin.

In the modified fibroin according to the present embodiment, it is preferred that the domain sequence has an amino acid sequence equivalent to an amino acid sequence in which, at least, in at least one motif sequence selected from GGX and GPGXX (where X represents an amino acid residue other than glycine) in REP, one glycine residue in one or a plurality of the motif sequences is substituted with another amino acid residue, as compared to the naturally occurring fibroin. By this configuration, the effect of the present invention is more significantly exhibited.

In the modified fibroin according to the present embodiment, it is more preferred that the ratio of the motif sequence in which the glycine residue is substituted with another amino acid residue is 10% or more with respect to the entire motif sequence. This makes it possible to more stably exert the effect of the present invention that the amount of production of the fibroin in the recombinant protein production system can be significantly improved without decreasing the elongation thereof.

The modified fibroin according to the present embodiment may further have modifications of an amino acid sequence corresponding to substitution, deletion, insertion and/or addition of one or a plurality of amino acid residues as compared to naturally occurring fibroin, in addition to the modification on the glycine residue in REP described above.

The modified fibroin according to the present embodiment can be obtained, for example, by substituting one or a plurality of glycine residues in REP with another amino acid residue, from the gene sequence of cloned naturally occurring fibroin. Further, for example, the modified fibroin according to the present embodiment can also be obtained by designing an amino acid sequence corresponding to substitution of one or a plurality of glycine residues in REP with another amino acid residue, from the amino acid sequence of naturally occurring fibroin, and chemically synthesizing a nucleic acid encoding the designed amino acid sequence. In any case, in addition to modification corresponding to substitution of one or a plurality of glycine residues in REP with another amino acid residue, from the amino acid sequence of naturally occurring fibroin, further modification of the amino acid sequence corresponding to substitution, deletion, insertion and/or addition of one or a plurality of amino acid residues may be carried out. Substitution, deletion, insertion and/or addition of amino acid residues can be carried out by methods well known to those skilled in the art, such as site-directed mutagenesis. Specifically, it can be carried out according to a method described in literatures such as Nucleic Acid Res. 10, 6487 (1982), and Methods in Enzymology, 100, 448 (1983).

Naturally occurring fibroin is a protein including a domain sequence represented by Formula 1: $[(A)_n$ motif-$REP]_m$, specifically, for example, a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and Hornet silk proteins discharged by larvae of Vespa simillima xanthoptera.

A more specific example of the fibroin produced by insects may be a silkworm fibroin L chain (GenBank Accession No. M76430 (nucleotide sequence), AAA27840.1 (amino acid sequence)).

Examples of the fibroin produced by spiders include spider silk proteins produced by spiders belonging to the genus *Araneus* such as *Araneus ventricosus, Araneus diadematus, Araneus pinguis, Araneus pentagrammicus* and *Araneus nojimai,* spiders belonging to the genus *Neoscona* such as *Neoscona scylla, Neoscona nautica, Neoscona adianta* and *Neoscona scylloides,* spiders belonging to the genus *Pronus* such as *Pronous minutes,* spiders belonging to the genus *Cyrtarachne* such as *Cyrtarachne bufo* and *Cyrtarachne inaequalis,* spiders belonging to the genus *Gasteracantha* such as *Gasteracantha kuhli* and *Gasteracantha mammosa,* spiders belonging to the genus *Ordgarius* such as *Ordgarius hobsoni* and *Ordgarius sexspinosus,* spiders belonging to the genus *Argiope* such as *Argiope amoena, Argiope minuta* and *Argiope bruennich,* spiders belonging to the genus *Arachnura* such as *Arachnura logio,* spiders belonging to the genus *Acusilas* such as *Acusilas coccineus,* spiders belonging to the genus *Cytophora* such as *Cyrtophora moluccensis, Cyrtophora exanthematica* and *Cyrtophora unicolor,* spiders belonging to the genus *Poltys* such as *Poltys illepidus,* spiders belonging to the genus *Cyclosa* such as *Cyclosa octotuberculata, Cyclosa sedeculata, Cyclosa vallata* and *Cyclosa atrata,* and spiders belonging to the genus *Chorizopes* such as *Chorizopes nipponicus;* and spider silk proteins produced by spiders belonging to the genus *Tetragnatha* such as *Tetragnatha praedonia, Tetragnatha maxillosa, Tetragnatha extensa* and *Tetragnatha squamata,* spiders belonging to the genus *Leucauge* such as *Leucauge magnifica, Leucauge blanda* and *Leucauge subblanda,* spiders belonging to the genus *Nephila* such as *Nephila clavata* and *Nephila pilipes,* spiders belonging to the genus *Menosira* such as *Menosira ornata,* spiders belonging to the genus *Dyschiriognatha* such as *Dyschiriognatha tenera,* spiders belonging to the genus *Latrodectus* such as *Latrodectus mactans, Latrodectus hasseltii, Latrodectus geometricus* and *Latrodectus tredecimguttatus,* and spiders belonging to the family *Tetragnathidae* such as spiders belonging to the genus *Euprosthenops.* Examples of spider silk proteins include traction fiber proteins such as MaSp (MaSp1 and MaSp2) and ADF (ADF3 and ADF4), and MiSp (MiSp1 and MiSp2).

More specific examples of the fibroin produced by spiders include fibroin-3 (adf-3) [derived from *Araneus diadematus*] (GenBank Accession Number AAC47010 (amino acid sequence), U47855 (nucleotide sequence)), fibroin-4 (adf-4) [derived from *Araneus diadematus*] (GenBank Accession Number AAC47011 (amino acid sequence), U47856 (nucleotide sequence)), dragline silk protein spidroin 1 [derived from *Nephila clavipes*] (GenBank Accession Number AAC04504 (amino acid sequence), U37520 (nucleotide sequence)), major angulate spidroin 1 [derived from *Latrodectus hesperus*] (GenBank Accession Number ABR68856 (amino acid sequence)), EF595246 (nucleotide sequence)), dragline silk protein spidroin 2 [derived from *Nephila clavata*] (GenBank Accession Number AAL32472 (amino acid sequence), AF441245 (nucleotide sequence)), major anpullate spidroin 1 [derived from *Euprosthenops australis*] (GenBank Accession Number CAJ00428 (amino acid sequence), AJ973155 (nucleotide sequence)) and major ampullate spidroin 2 [*Euprosthenops australis*] (GenBank Accession Number CAM32249.1 (amino acid sequence), AM490169 (nucleotide sequence)), minor ampullate silk protein 1 [*Nephila clavipes*] (GenBank Accession Number AAC14589.1 (amino acid sequence)), minor ampullate silk protein 2 [*Nephila clavipes*] (GenBank Accession Number AAC14591.1 (amino acid sequence)), and minor ampullate spidroin-like protein [*Nephilengys cruentata*] (GenBank Accession Number ABR37278.1 (amino acid sequence)).

As a more specific example of naturally occurring fibroin, fibroin in which sequence information is registered in NCBI GenBank can be further mentioned. For example, it can be confirmed by extracting sequences in which spidroin, ampullate, fibroin, "silk and polypeptide", or "silk and protein" is described as a keyword in DEFINITION among sequences containing INV as DIVISION among sequence information registered in NCBI GenBank, sequences in which a specific character string of products is described from CDS, or sequences in which a specific character string is described from SOURCE to TISSUE TYPE.

The modified fibroin according to another embodiment includes a domain sequence represented by Formula 1: $[(A)_n$ motif-$REP]_m$, and has an amino acid sequence in which z/w is 50.9% or more in the case where the total number of amino acid residues in the amino acid sequence consisting of XGX (where X represents an amino acid residue other than glycine) contained in all REPs in the sequence excluding the sequence from the $(A)_n$ motif located at the most C-terminal side to the C-terminal of the domain sequence from the domain sequence is defined as z, and the total number of amino acid residues in the sequence excluding the sequence from the $(A)_n$ motif located at the most C-terminal side to the C-terminal of the domain sequence from the domain sequence is defined as w. Since the content of glycine residues in the modified fibroin according to the present embodiment is reduced, the ratio at which the value of z/w corresponding to the content ratio of the amino acid sequence consisting of XGX falls within the above-specified range is high.

In the modified fibroin according to the present embodiment, it is preferable to increase the content ratio of the amino acid sequence consisting of XGX by substituting one glycine residue of the GGX motif with another amino acid residue. In the modified fibroin according to the present embodiment, the content ratio of the amino acid sequence consisting of GGX in the domain sequence is preferably 6% or less, more preferably 4% or less, and still more preferably 2% or less. The content ratio of the amino acid sequence consisting of GGX in the domain sequence can be calculated by the same method as the calculation method of the content ratio (z/w) of the amino acid sequence consisting of XGX described below.

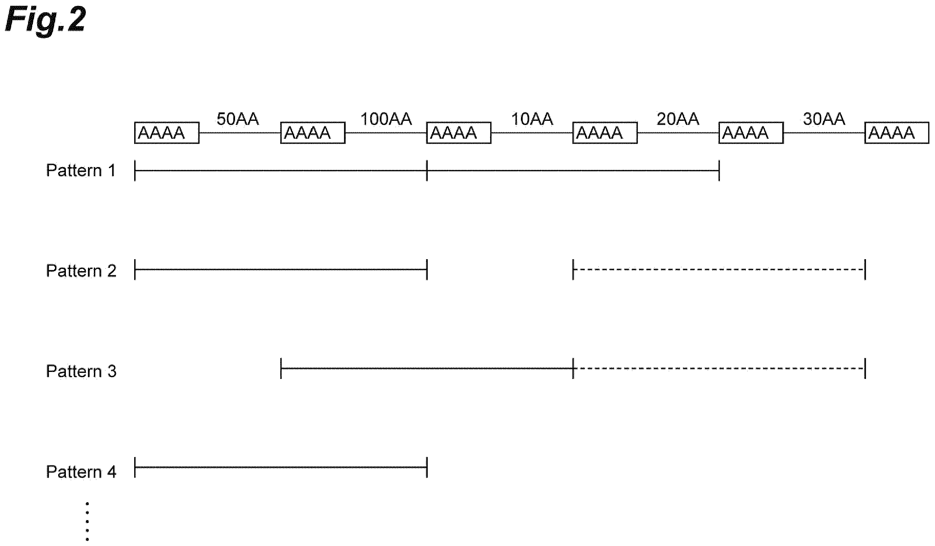

The calculation method of z/w will be described in more detail. First, an amino acid sequence consisting of XGX is extracted from all the REPs contained in the sequence excluding the sequence from the $(A)_n$ motif located at the most C-terminal side to the C-terminal of the domain sequence from the domain sequence. The total number of amino acid residues constituting XGX is z. For example, in the case where 50 amino acid sequences consisting of XGX are extracted (there is no overlap), z is $50 \times 3 = 150$. Also, for example, in the case where X (central X) contained in two XGXs exists as in the case of the amino acid sequence consisting of XGXGX, it is calculated by subtracting the overlapping portion (in the case of XGXGX, it is 5 amino acid residues). w is the total number of amino acid residues contained in the sequence excluding the sequence from the $(A)_n$ motif located at the most C-terminal side to the C-terminal of the domain sequence from the domain sequence. For example, in the case of the domain sequence shown in FIG. 2, w is 4+50+4+100+4+10+4+20+4+30=230 (excluding the $(A)_n$ motif located at the most C-terminal side). Next, z/w (%) can be calculated by dividing z by w.

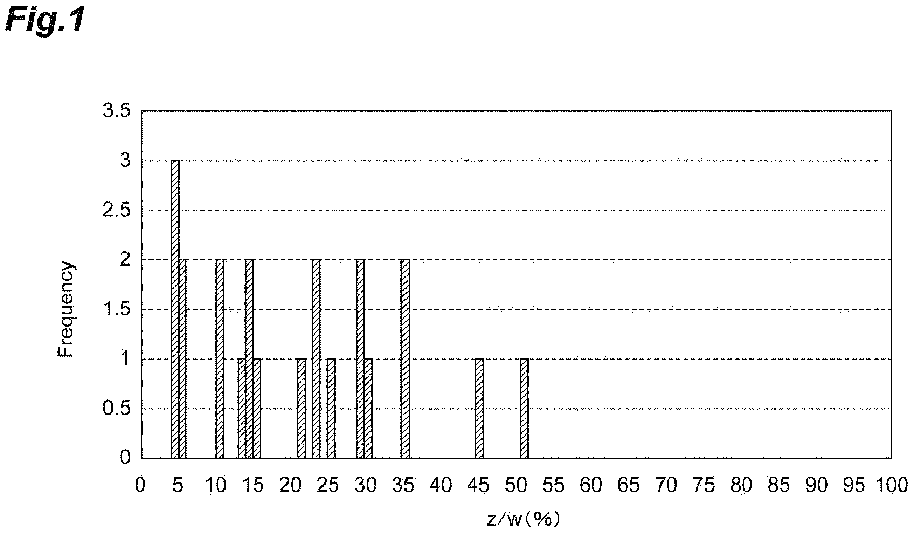

Here, z/w in naturally occurring fibroin will be described. First, as described above, 663 types of fibroins (415 types of fibroins derived from spiders among them) were extracted by confirming fibroins with amino acid sequence information registered in NCBI GenBank by a method exemplified. z/w was calculated by the above-mentioned calculation method from the amino acid sequences of naturally occurring fibroins which include a domain sequence represented by Formula 1: $[(A)_n \text{ motif-REP}]_m$ and in which the content ratio of the amino acid sequence consisting of GGX in the fibroin is 6% or less, among all the extracted fibroins. The results are shown in FIG. 1. In FIG. 1, the horizontal axis represents z/w (%) and the vertical axis represents frequency. As is apparent from FIG. 1, z/w in naturally occurring fibroin is less than 50.9% (highest, 50.86%).

In the modified fibroin according to the present embodiment, z/w is preferably 50.9% or more, more preferably 56.1% or more, still more preferably 58.7% or more, even still more preferably 70% or more, and still further preferably 80% or more. The upper limit of z/w is not particularly limited, but it may be 95% or less, for example.

The modified fibroin according to the present embodiment can be obtained, for example, by substituting and modifying at least a part of a nucleotide sequence encoding a glycine residue from the gene sequence of cloned naturally occurring fibroin so as to encode another amino acid residue. At this time, one glycine residue in the GGX motif and GPGXX motif may be selected as the glycine residue to be modified, and substitution may be carried out such that z/w is 50.9% or more. Alternatively, the modified fibroin according to the present embodiment can also be obtained, for example, by designing an amino acid sequence satisfying each of the above embodiments from the amino acid sequence of naturally occurring fibroin and chemically synthesizing a nucleic acid encoding the designed amino acid sequence. In any case, in addition to the modification corresponding to substitution of a glycine residue in REP with another amino acid residue from the amino acid sequence of naturally occurring fibroin, modification of the amino acid sequence corresponding to substitution, deletion, insertion and/or addition of one or a plurality of amino acid residues may be carried out.

The above-mentioned another amino acid residue is not particularly limited as long as it is an amino acid residue other than a glycine residue, but it is preferably a hydrophobic amino acid residue such as a valine (V) residue, a leucine (L) residue, an isoleucine (I) residue, a methionine (M) residue, a proline (P) residue, a phenylalanine (F) residue, or a tryptophan (W) residue, or a hydrophilic amino acid residue such as a glutamine (Q) residue, an asparagine (N) residue, a serine (S) residue, a lysine (K) residue, or a glutamic acid (E) residue, among which more preferred is a valine (V) residue, a leucine (L) residue, an isoleucine (I) residue or a glutamine (Q) residue, and still more preferred is a glutamine (Q) residue.

In the modified fibroin of the present invention, it is preferred that the domain sequence has an amino acid sequence in which the content of the $(A)_n$ motif is reduced in addition to having a reduced content of glycine residues as compared to naturally occurring fibroin. By this configuration, the effect of the present invention is more significantly exhibited. The domain sequence of the modified fibroin can be said to have an amino acid sequence equivalent to an amino acid sequence in which one or a plurality of $(A)_n$ motifs is deleted, as well as at least one or a plurality of glycine residues in REP is substituted with another amino acid residue, as compared to naturally occurring fibroin.

Next, a specific embodiment of the domain sequence in which the content of the $(A)_n$ motif is reduced will be described.

Although the description on the reduction of the content of glycine residues is omitted, each of the above embodiments relating to the reduction of the content of glycine residues and each of following embodiments relating to the reduction of the content of the $(A)_n$ motif can be arbitrarily combined.

In the modified fibroin according to one embodiment, the domain sequence has an amino acid sequence equivalent to an amino acid sequence in which, at least, one $(A)_n$ motif per one to three $(A)_n$ motifs from the N-terminal side to the C-terminal side is deleted, as compared to naturally occurring fibroin.

In the modified fibroin according to another embodiment, the domain sequence has an amino acid sequence equivalent to an amino acid sequence in which, at least, two consecutive $(A)_n$ motif deletions and one $(A)_n$ motif deletion are repeated in this order from the N-terminal side to the C-terminal side, as compared to the naturally occurring fibroin.

In the modified fibroin according to yet another embodiment, the domain sequence has an amino acid sequence equivalent to an amino acid sequence in which, at least, $(A)_n$ motif every other two positions is deleted from the N-terminal side to the C-terminal side.

Since the $(A)_n$ motif of fibroin was thought to be closely related to the strength (stress and toughness) of fibroin, research and development have been advanced to increase the content of $(A)_n$ motif so far, and it has been thought that the strength is significantly decreased by decreasing the content of $(A)_n$ motif. However, the present inventors have found that, even in the case where the content of the $(A)_n$ motif is decreased, the stress does not decrease significantly, the amount of production in the recombinant protein production system can be significantly improved, and further the toughness and elongation are also improved. According to the modified fibroin of the present embodiment, such an unexpected effect is also exerted.

In the present embodiment, the domain sequence of the modified fibroin may have an amino acid sequence equivalent to an amino acid sequence in which 10 to 40% of the $(A)_n$ motif is deleted from naturally occurring fibroin. In the case where the decrease in the content of the $(A)_n$ motif is within this range, it is possible to stably exert the effects that the amount of production of fibroin in the recombinant protein production system can be significantly improved without significantly reducing the stress, and the toughness and elongation can also be improved.

In still another embodiment, the modified fibroin has an amino acid sequence in which a maximum value of x/y is 20% or more, in the case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the total value of the number of amino acid residues in the two adjacent $[(A)_n$ motif-REP] units where the ratio of the number of amino acid residues in the other REP is 2 to 3.5 is defined as x, and the total number of amino acid residues of the domain sequence is y.

A method of calculating x/y will be described in more detail with reference to FIG. 2. FIG. 2 shows a domain sequence excluding N-terminal sequence and C-terminal sequence from modified fibroin. This domain sequence has a sequence of $(A)_n$ motif-first REP (50 amino acid residues)-$(A)_n$ motif-second REP (100 amino acid residues)-$(A)_n$ motif-third REP (10 amino acid residues)-$(A)_n$ motif-fourth REP (20 amino acid residues)-$(A)_n$ motif-fifth REP (30 amino acid residues)-$(A)_n$ motif from the N-terminal side (left side).

The two adjacent $[(A)_n$ motif-REP] units are sequentially selected from the N-terminal side to the C-terminal side so as not to overlap. At this time, an unselected $[(A)_n$ motif-REP] unit may exist. FIG. 2 shows a pattern 1 (a comparison between first REP and second REP and a comparison between third REP and fourth REP), a pattern 2 (a comparison between first REP and second REP and a comparison between fourth REP and fifth REP), a pattern 3 (a comparison between second REP and third REP and a comparison between fourth REP and fifth REP), and a pattern 4 (a comparison between first REP and second REP). There are other selection methods besides this.

Next, for each pattern, the number of amino acid residues of each REP in the selected two adjacent $[(A)_n$ motif-REP] units is compared. The comparison is carried out by obtaining the ratio of the number of amino acid residues of the other REP in the case where one REP having a smaller number of amino acid residues is 1. For example, in the case of comparing the first REP (50 amino acid residues) and the second REP (100 amino acid residues), the ratio of the number of amino acid residues of the second REP is 100/50 = 2 in the case where the first REP having a smaller number of amino acid residues is 1. Similarly, in the case of comparing the fourth REP (20 amino acid residues) and the fifth REP (30 amino acid residues), the ratio of the number of amino acid residues of the fifth REP is 30/20 = 1.5 in the case where the fourth REP having a smaller number of amino acid residues is 1.

In FIG. 2, a set of $[(A)_n$ motif-REP] units in which the ratio of the number of amino acid residues of the other REP is 1.8 to 11.3 in the case where one REP having a smaller number of amino acid residues is 1 is indicated by a solid line. Hereinafter, such a ratio is referred to as a Giza ratio. A set of $[(A)_n$ motif-REP] units in which the ratio of the number of amino acid residues of the other REP is less than 1.8 or more than 11.3 in the case where one REP having a smaller number of amino acid residues is 1 is indicated by a broken line.

In each pattern, the number of all amino acid residues of two adjacent $[(A)_n$ motif-REP] units indicated by solid lines (including not only the number of amino acid residues of REP but also the number of amino acid residues of $(A)_n$ motif) is combined. Then, the total values thus combined are compared and the total value of the pattern whose total value is the maximum (the maximum value of the total value) is defined as x. In the example shown in FIG. 2, the total value of the pattern 1 is the maximum.

Next, x/y (%) can be calculated by dividing x by the total amino acid residue number y of the domain sequence.

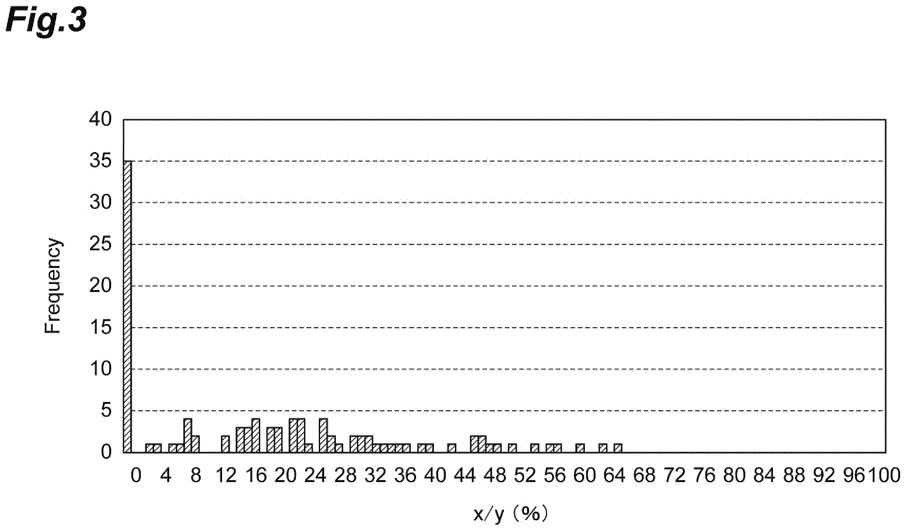

Here, x/y in naturally occurring fibroin will be described. First, as described above, 663 types of fibroins (415 types of fibroins derived from spiders among them) were extracted by confirming fibroins with amino acid sequence information registered in NCBI GenBank by a method exemplified. x/y was calculated by the above-mentioned calculation method from the amino acid sequences of naturally occurring fibroins which include a domain sequence represented by Formula 1: $[(A)_n \text{ motif-REP}]_m$ and in which at least seven of a plurality of the $(A)_n$ motifs in the domain sequence consist of only alanine residues, among all the extracted fibroins. FIG. 3 shows the results in the case where the Giza ratio is 1:1.9 to 4.1. In FIG. 3, the horizontal axis represents x/y (%) and the vertical axis represents frequency. x/y in naturally occurring fibroin was less than 64.2% (highest, 64.14%). In the naturally occurring fibroin which includes a domain sequence represented by Formula 1: $[(A)_n \text{ motif-REP}]_m$ and in which at least seven of a plurality of the $(A)_n$ motifs in the domain sequence consist of only alanine residues (as described above, z/w in naturally occurring fibroin is less than 46.4%), the effect is recognized in the case where x/y is 20% or more.

In the modified fibroin according to the present embodiment, x/y may be 20% or more. x/y is preferably 40% or more, more preferably 50% or more, still more preferably 60% or more, even still more preferably 64.2% or more, still further preferably 70% or more, particularly preferably 75% or more, and most preferably 80% or more. The upper limit of x/y is not particularly limited, and it may be 100% or less.

A modified fibroin including a domain sequence with a reduced $(A)_n$ motif content can be obtained, for example, from a gene sequence of cloned naturally occurring fibroin, by deleting one or a plurality of the sequences encoding the $(A)_n$ motif such that x/y is 20% or more. Further, the modified fibroin including a domain sequence with a reduced $(A)_n$ motif content can also be obtained, for example, by designing an amino acid sequence corresponding to deletion of one or a plurality of $(A)_n$ motifs such that x/y is 20% or more, from the amino acid sequence of naturally occurring fibroin, and chemically synthesizing a nucleic acid encoding the designed amino acid sequence.

A more specific example of the modified fibroin according to the present invention may be a modified fibroin including (i) an amino acid sequence set forth in SEQ ID NO: 3, SEQ ID NO: 4 or SEQ ID NO: 10, or (ii) an amino acid sequence having 90% or more sequence identity with the amino acid sequence set forth in SEQ ID NO: 3, SEQ ID NO: 4 or SEQ ID NO: 10.

The modified fibroin of (i) will be described. The amino acid sequence set forth in SEQ ID NO: 3 is the amino acid sequence in which all GGX in REP of the amino acid sequence set forth in SEQ ID NO: 1, which corresponds to naturally occurring fibroin, is substituted with GQX. The amino acid sequence set forth in SEQ ID NO: 4 is the amino acid sequence in which $(A)_n$ motif every other two positions from the N-terminal side to the C-terminal side are deleted from the amino acid sequence set forth in SEQ ID NO: 3 and further one $[(A)_n \text{ motif-REP}]$ is inserted before the C-terminal sequence. The amino acid sequence set forth in SEQ ID NO: 10 is the amino acid sequence in which two alanine residues are inserted at the C-terminal side of each $(A)_n$ motif of the amino acid sequence set forth in SEQ ID NO: 4 and further a part of glutamine (Q) residues is substituted with a serine (S) residue and a part of amino acids on the N-terminal side is deleted so as to be almost the same as the molecular weight of SEQ ID NO: 4. In addition, the amino acid sequence set forth in SEQ ID NO: 2 is the amino acid sequence in which $(A)_n$ motif every other two positions from the N-terminal side to the C-terminal side are deleted from the amino acid sequence set forth in SEQ ID NO: 1.

The value of z/w in the amino acid sequence set forth in SEQ ID NO: 1 (corresponding to naturally occurring fibroin) is 46.8% (see Table 1). The values of z/w in the amino acid sequence set forth in SEQ ID NO: 3, the amino acid sequence set forth in SEQ ID NO: 4, and the amino acid sequence set forth in SEQ ID NO: 10 are respectively 58.7%, 70.1% and 66.1% (See Table 1). In addition, the values of x/y at the Giza ratio 1:1.8 to 11.3 of the amino acid sequences set forth in SEQ ID NOs: 1, 3, 4 and 10 are respectively 15.0%, 15.0%, 93.4% and 92.7% (see Tables 2 to 5).

[Table 1]

| Origin | Number of XGX | Overlapping AA | Total number (z) of amino acid residues constituting XGX | Total value (w) of amino acid residues in domain | z/w (%) |
|---|---|---|---|---|---|
| Met-PRT313 (SEQ ID NO: 1) | 97 | 26 | 265 | 566 | 46.8 |
| Met-PRT380 (SEQ ID NO: 3) | 127 | 49 | 332 | 566 | 58.7 |
| Met-PRT410 (SEQ ID NO: 4) | 152 | 64 | 392 | 559 | 70.1 |
| Met-PRT468 (SEQ ID NO: 10) | 137 | 58 | 353 | 534 | 66.1 |

[Table 2]

| Met-PRT313 (SEQ ID NO: 1) | Number of residues in unit | Number of residues in REP | Pattern 1 | | Pattern 2 | | Pattern 3 | |
|---|---|---|---|---|---|---|---|---|
| | | | Ratio of number of residues | Total number of residues in two units | Ratio of number of residues | Total number of residues in two units | Ratio of number of residues | Total number of residues in two units |
| MGPGGQGPYGPG (N-terminal sequence) | - | - | - | - | - | - | - | - |
| ASAAAAAGGNGPGSGQQGPGGS | 22 | 15 | 1.2 | - | 1.2 | - | 1.4 | - |
| AAAAAGGYGPGGQGPGQQGPGSS | 23 | 18 | | | | | | |
| AAAAAGPGGYGPGGQGPS | 18 | 13 | 1.3 | - | - | - | | |
| ASAAAAAGPGSGQQGPG | 17 | 10 | | | 1.8 | 42 | 1.8 | 42 |
| ASAAAAAGGYGPGGQGPGQQGPGSS | 25 | 18 | 1.2 | - | | | | |
| AAAAAGGYGSGPGQQGPYGS | 20 | 15 | | | 1.0 | - | 1.0 | - |
| AAAAAGPGSGGYGQGPYGPG | 20 | 15 | 1.2 | - | | | | |
| ASAAAAAGPGGYGPGGQGPS | 20 | 13 | | | 1.0 | - | 1.0 | - |
| ASAAAAAGSGQQGPGGYGPY | 20 | 13 | 1.4 | - | | | | |
| ASAAAAAGGYGSGPGQQGPYGPGGS | 25 | 18 | | | 1.4 | - | 1.4 | - |
| AAAAAGSGQQGPGQQGPY | 18 | 13 | 1.0 | - | | | | |
| ASAAAAAGPGGQGPYGPGSS | 20 | 13 | | | 1.2 | - | 1.2 | - |
| AAAAAGGYGYGPGGQGPYGPG | 21 | 16 | 1.3 | - | | | | |
| ASAAAAAGGNGPGSGGYGPGQQGPGGS | 27 | 20 | | | 1.8 | 43 | 1.8 | 43 |
| AAAAAGPGGQGPYGPG | 16 | 11 | 1.6 | - | | | | |
| ASAAAAAGGYGPGGQGPGGYGPGSS | 25 | 18 | | | 1.4 | - | 1.4 | - |
| AAAAAGPGGQGPYGPGSS | 18 | 13 | 1.2 | - | | | | |
| AAAAAGGYGPGQQGPYGPGGS | 21 | 16 | | | 1.0 | - | 1.0 | - |
| AAAAAGGYQQGPGGQGPYGPG | 21 | 16 | 1.5 | - | | | | |
| ASAAAAAGPGGQGPYGPG | 18 | 11 | | | 1.2 | - | 1.2 | - |
| ASAAAAAGPGGYGPGGQGPS | 20 | 13 | 1.4 | - | | | | |
| ASAAAAAGGYGSGPGGYGPYGPGGS | 25 | 18 | | | 1.2 | - | 1.2 | - |
| AAAAAGPGSGQQGQGPYGPG | 20 | 15 | 1.1 | - | | | | |
| ASAAAAAGGYGPGQQGPYGPGGS | 23 | 16 | | | 1.6 | - | 1.6 | - |
| AAAAAGPGSGGYGPG | 15 | 10 | 2.0 | 42 | | | | |
| ASAAAAAGGNGPGSGGYGPGQQGPGGS | 27 | 20 | | | 1.3 | - | 1.3 | - |
| AAAAAGGYQQGPGGQGPYGPG | 21 | 16 | | | | | | |
| ASAAAAAGPGSGQQGPGAS (C-terminal sequence) | - | - | - | - | - | - | - | - |
| | | | Total number of residues at ratio of 1.8 to 11.3 (x1) | 42 | Total number of residues at ratio of 1.8 to 11.3 (x2) | 85 | Total number of residues at ratio of 1.8 to 11.3 (x3) | 85 |
| Total number of amino acid residues in domain sequence (y) = | 566 | | x1/y= | 7.4% | x2/y= | 15.0% | x3/y= | 15.0% |

[Table 3]

| Met-PRT380 (SEQ ID NO: 3) | Number of residues in unit | Number of residues in REP | Pattern 1 — Ratio of number of residues | Pattern 1 — Total number of residues in two units | Pattern 2 — Ratio of number of residues | Pattern 2 — Total number of residues in two units | Pattern 3 — Ratio of number of residues | Pattern 3 — Total number of residues in two units |
|---|---|---|---|---|---|---|---|---|
| MGPGQQGPYGPG (N-terminal sequence) | - | - | - | - | - | - | - | - |
| ASAAAAAGQNGPGSGQQGPGQS | 22 | 15 | 1.2 | - | 1.2 | - | 1.4 | - |
| AAAAAGQYGPGQQGPGQQGPGSS | 23 | 18 | 1.3 | - | 1.8 | 42 | 1.8 | 42 |
| AAAAAGPGQYGPGQQGPS | 18 | 13 | | - | 1.0 | - | 1.0 | - |
| ASAAAAAGPGSGQQGPG | 17 | 10 | 1.2 | - | 1.0 | - | 1.0 | - |
| ASAAAAAGQYGPGQQGPGQQGPGSS | 25 | 18 | 1.2 | - | 1.4 | - | 1.4 | - |
| AAAAAGQYGSGPGQYGQGPYGS | 20 | 15 | | - | 1.2 | - | 1.2 | - |
| AAAAAGPGSGQYGQGPYGPG | 20 | 15 | 1.4 | - | 1.8 | 43 | 1.8 | 43 |
| ASAAAAAGPGQYGPGQQGPS | 20 | 13 | | - | 1.4 | - | 1.4 | - |
| ASAAAAAGSGQQGPGQYGPY | 20 | 13 | 1.0 | - | 1.0 | - | 1.0 | - |
| ASAAAAAGQYGSGPGQQGPYGPGQS | 25 | 18 | | - | 1.2 | - | 1.2 | - |
| AAAAGSGQQGPGQQGPY | 18 | 13 | 1.3 | - | 1.2 | - | 1.2 | - |
| ASAAAAAGPGQQGPYGPGSS | 20 | 13 | | - | 1.6 | - | 1.6 | - |
| ASAAAAAGQNGPGSGQYGPGQQGPGQS | 27 | 20 | 1.6 | - | 1.3 | - | 1.3 | - |
| AAAAAGPGQQGPYGPG | 16 | 11 | | - | | - | | - |
| ASAAAAAGQYGPGQQGPGQYGPGSS | 25 | 18 | 1.2 | - | | - | | - |
| AAAAAGPGSGQQGPYGPGSS | 18 | 13 | | - | | - | | - |
| AAAAAGQYGPGQQGPYGPG | 21 | 16 | 1.5 | - | | - | | - |
| AAAAAGPGQQGPYGPG | 18 | 11 | | - | | - | | - |
| ASAAAAAGPGQYGPGQQGPS | 20 | 13 | 1.4 | - | | - | | - |
| ASAAAAAGQYGSGPGQYGPYGPGQS | 25 | 18 | | - | | - | | - |
| AAAAAGPGSGQQGPYGPG | 20 | 15 | 1.1 | - | | - | | - |
| ASAAAAAGQYGPGQQGPYGPGQS | 23 | 16 | | - | | - | | - |
| AAAAAGPGSGQYGPG | 15 | 10 | 2.0 | 42 | | - | | - |
| ASAAAAAGQNGPGSGQYGPGQQGPGQS | 27 | 20 | | - | | - | | - |
| AAAAAGQYQQGPGQQGPYGPG | 21 | 16 | | - | | - | | - |
| ASAAAAAGPGSGQQGPGAS (C-terminal sequence) | - | - | - | - | - | - | - | - |
| | | | Total number of residues at ratio of 1.8 to 11.3 (x1) | 42 | Total number of residues at ratio of 1.8 to 11.3 (x2) | 85 | Total number of residues at ratio of 1.8 to 11.3 (x3) | 85 |
| Total number of amino acid residues in domain sequence (y) = | 566 | - | x1/y= | 7.4% | x2/y= | 15.0% | x3/y= | 15.0% |

[Table 4]

| Met-PRT410 (SEQ ID NO: 4) | Number of residues in unit | Number of residues in REP | Pattern 1 | | Pattern 2 | | Pattern 3 | |
|---|---|---|---|---|---|---|---|---|
| | | | Ratio of number of residues | Total number of residues in two units | Ratio of number of residues | Total number of residues in two units | Ratio of number of residues | Total number of residues in two units |
| MGPGQQGPYGPG (N-terminal sequence) | - | - | - | - | - | - | - | - |
| ASAAAAAGQNGPGSGQQGPGQSGQYGPGQQGPGQQGPGSS | 40 | 33 | 2.5 | 58 | 2.5 | 58 | | |
| AAAAAGPGQYGPGQQGPS | 18 | 13 | | | | | 2.3 | 55 |
| ASAAAAAGPGSGQQGPGASGQYGPGQQGPGQQGPGSS | 37 | 30 | 2.0 | 57 | - | - | | |
| AAAAAGQYGSGPGQQGPYGS | 20 | 15 | | | 2.0 | 55 | 2.0 | 55 |
| AAAAAGPGSGQYGQGPYGPGASGPGQYGPGQQGPS | 35 | 30 | 2.3 | 55 | | | | |
| ASAAAAAGSGQQGPGQYGPY | 20 | 13 | | | 2.4 | 58 | 2.4 | 58 |
| ASAAAAAGQYGSGPGQQGPYGPGQSGSGQQGPGQQGPY | 38 | 31 | 2.4 | 58 | | | | |
| ASAAAAAGPGQQGPYGPGSS | 20 | 13 | | | 2.9 | 63 | 2.9 | 63 |
| AAAAAGQYGYGPGQQGPYGPGASGQNGPGSGQYGPGQQGPGQS | 43 | 38 | 3.5 | 59 | | | | |
| AAAAAGPGQQGPYGPG | 16 | 11 | | | 2.8 | 54 | 2.8 | 54 |
| ASAAAAAGQYGPGQQGPGQYGPGSSGPGQQGPYGPGSS | 38 | 31 | 1.9 | 59 | | | | |
| AAAAAGQYGPGQQGPYGPGQS | 21 | 16 | | | 1.8 | 55 | 1.8 | 55 |
| AAAAAGQYQQGPGQQGPYGPGASGPGQQGPYGPG | 34 | 29 | 2.2 | 54 | | | | |
| ASAAAAAGPGQYGPGQQGPS | 20 | 13 | | | 2.5 | 60 | 2.5 | 60 |
| ASAAAAAGQYGSGPGQYGPYGPGQSGPGSGQQGQGPYGPG | 40 | 33 | 2.1 | 63 | | | | |
| ASAAAAAGQYGPGQQGPYGPGQS | 23 | 16 | | | 2.0 | 60 | 2.0 | 60 |
| AAAAAGPGSGQYGPGASGQNGPGSGQYGPGQQGPGQS | 37 | 32 | 2.0 | 58 | | | | |
| AAAAAGQYQQGPGQQGPYGPG | 21 | 16 | | | 1.9 | 59 | 1.9 | 59 |
| ASAAAAAGQYGSGPGQQGPYGPGQSGSGQQGPGQQGPY | 38 | 31 | - | - | | | | |
| ASAAAAAGPGSGQQGPGAS (C-terminal sequence) | - | - | | | - | - | - | - |
| | | | Total number of residues at ratio of 1.8 to 11.3 (x1) | 521 | Total number of residues at ratio of 1.8 to 11.3 (x2) | 522 | Total number of residues at ratio of 1.8 to 11.3 (x3) | 519 |
| Total number of amino acid residues in domain sequence (y) = | 559 | | x1/y= | 93.2% | x2/y= | 93.4% | x3/y= | 92.8% |

EP 3 450 451 A1

14

[Table 5]

| Met-PRT468 (SEQ ID NO: 10) | Number of residues in unit | Number of residues in REP | Pattern 1 — Ratio of number of residues | Pattern 1 — Total number of residues in two units | Pattern 2 — Ratio of number of residues | Pattern 2 — Total number of residues in two units | Pattern 3 — Ratio of number of residues | Pattern 3 — Total number of residues in two units |
|---|---|---|---|---|---|---|---|---|
| MGPGQQGPYGPG (N-terminal sequence) | - | - | - | - | - | - | - | - |
| ASAAAAAAGSNGPGSGQQGPGQSGQYGPGQQGPGQQGPGSS | 42 | 33 | 2.5 | 62 | 2.5 | 62 | - | - |
| AAAAAAGPGQYGPGQQGPS | 20 | 13 | - | - | - | - | 2.3 | 59 |
| ASAAAAAAGPGSGQQGPGASGQYGPGQQGPGQQGPGSS | 39 | 30 | 2.0 | 61 | - | - | - | - |
| AAAAAAGSYGSGPGQQGPYGS | 22 | 15 | - | - | 2.0 | 59 | 2.0 | 59 |
| AAAAAAGPGSGQYGPGYGPGASGPGQYGPGPGQQGPS | 37 | 30 | 2.3 | 59 | - | - | - | - |
| ASAAAAAAGSGQQGPGQYGPY | 22 | 13 | - | - | 2.4 | 62 | 2.4 | 62 |
| ASAAAAAAGSYGSGPGQQGPYGPGQSGSGQQGPGQQGPY | 40 | 31 | 2.4 | 62 | - | - | - | - |
| ASAAAAAAGPGQQGPYGPGSS | 22 | 13 | - | - | 2.9 | 67 | 2.9 | 67 |
| AAAAAAGSYGYGPGQQGPYGPGASGQNGPGSGQYGPGQQGPGPS | 45 | 38 | 3.5 | 63 | - | - | - | - |
| AAAAAAGPGQQGPYGPG | 18 | 11 | - | - | 2.8 | 58 | 2.8 | 58 |
| ASAAAAAAGSYGPGQQGPGQYGPGSSGPGQQGPYGPGSS | 40 | 31 | 1.9 | 63 | - | - | - | - |
| AAAAAAGSYGPGQQGPYGPGPS | 23 | 16 | - | - | 1.8 | 59 | 1.8 | 59 |
| AAAAAAGSYQQGPGQQGPYGPGASGPGQQGPYGPG | 36 | 29 | 2.2 | 58 | - | - | - | - |
| ASAAAAAAGPGQYGPGQQGPS | 22 | 13 | - | - | 2.5 | 64 | 2.5 | 64 |
| ASAAAAAZLAGSYGSGPGQYGPYGPGQSGPGSGQQGQQGPYGPG | 42 | 33 | 2.1 | 67 | - | - | - | - |
| AAAAAAAGSYGPGQQGPYGPGPS | 25 | 16 | - | - | 2.0 | 64 | 2.0 | 64 |
| AAAAAAGSGQYGPGASGQNGPGSGQYGPGQQGPGPS | 39 | 32 | - | - | - | - | - | - |
| AAAAAAGPGSGQQGPGAS (C-terminal sequence) | - | - | - | - | - | - | - | - |
| | 534 | | Total number of residues at ratio of 1.8 to 11.3 (x1): 495 | | Total number of residues at ratio of 1.8 to 11.3 (x2): 495 | | Total number of residues at ratio of 1.8 to 11.3 (x3): 492 | |
| | | | x1/y= 92.7% | | x2/y= 92.7% | | x3/y= 92.1% | |

Total number of amino acid residues in domain sequence (y) = 534

The modified fibroin of (i) may consist of the amino acid sequence set forth in SEQ ID NO: 3, SEQ ID NO: 4 or SEQ ID NO: 10.

The modified fibroin of (ii) includes an amino acid sequence having 90% or more sequence identity with the amino acid sequence set forth in SEQ ID NO: 3, SEQ ID NO: 4 or SEQ ID NO: 10. The modified fibroin of (ii) is also a protein including a domain sequence represented by Formula 1: $[(A)_n$ motif-REP$]_m$. The sequence identity is preferably 95% or more.

It is preferred that the modified fibroin of (ii) has 90% or more sequence identity with the amino acid sequence set forth in SEQ ID NO: 3, SEQ ID NO: 4 or SEQ ID NO: 10, and z/w is 50.9% or more in the case where the total number of amino acid residues in the amino acid sequence consisting of XGX (where X represents an amino acid residue other than glycine) contained in REP is defined as z, and the total number of amino acid residues of REP in the domain sequence is defined as w.

The above-mentioned modified fibroin may include a tag sequence at either or both of the N-terminal and C-terminal. This makes it possible to isolate, immobilize, detect and visualize the modified fibroin.

The tag sequence may be, for example, an affinity tag utilizing specific affinity (binding property, affinity) with another molecule. As a specific example of the affinity tag, a histidine tag (His tag) can be mentioned. The His tag is a short peptide in which about 4 to 10 histidine residues are arranged and has a property of specifically binding to a metal ion such as nickel, so it can be used for isolation of modified fibroin by chelating metal chromatography. A specific example of the tag sequence may be an amino acid sequence set forth in SEQ ID NO: 5 (amino acid sequence including His tag).

In addition, a tag sequence such as glutathione-S-transferase (GST) that specifically binds to glutathione or a maltose binding protein (MBP) that specifically binds to maltose can also be used.

Further, an "epitope tag" utilizing an antigen-antibody reaction can also be used. By adding a peptide (epitope) showing antigenicity as a tag sequence, an antibody against the epitope can be bound. Examples of the epitope tag include an HA (peptide sequence of hemagglutinin of influenza virus) tag, a myc tag, and a FLAG tag. The modified fibroin can easily be purified with high specificity by utilizing an epitope tag.

It is also possible to use a tag sequence which can be cleaved with a specific protease. By treating a protein adsorbed through the tag sequence with protease, it is also possible to recover the modified fibroin cleaved from the tag sequence.

A more specific example of the modified fibroin including a tag sequence may be a modified fibroin including (iii) an amino acid sequence set forth in SEQ ID NO: 8, SEQ ID NO: 9 or SEQ ID NO: 11, or (iv) an amino acid sequence having 90% or more sequence identity with the amino acid sequence set forth in SEQ ID NO: 8, SEQ ID NO: 9 or SEQ ID NO: 11.

The amino acid sequences set forth in SEQ ID NOs: 6, 7, 8, 9 and 11 are amino acid sequences in which an amino acid sequence set forth in SEQ ID NO: 5 (including a His tag) is added at the N-terminals of the amino acid sequences set forth in SEQ ID NOs: 1, 2, 3, 4 and 10, respectively.

The modified fibroin of (iii) may consist of an amino acid sequence set forth in SEQ ID NO: 8, SEQ ID NO: 9 or SEQ ID NO: 11.

The modified fibroin of (iv) includes an amino acid sequence having 90% or more sequence identity with the amino acid sequence set forth in SEQ ID NO: 8, SEQ ID NO: 9 or SEQ ID NO: 11. The modified fibroin of (iv) is also a protein including a domain sequence represented by Formula 1: $[(A)_n$ motif-REP$]_m$. The sequence identity is preferably 95% or more.

It is preferred that the modified fibroin of (iv) has 90% or more sequence identity with the amino acid sequence set forth in SEQ ID NO: 8, SEQ ID NO: 9 or SEQ ID NO: 11, and z/w is 50.9% or more in the case where the total number of amino acid residues in the amino acid sequence consisting of XGX (where X represents an amino acid residue other than glycine) contained in REP is defined as z, and the total number of amino acid residues of REP in the domain sequence is defined as w.

The above-mentioned modified fibroin may include a secretory signal for releasing the protein produced in the recombinant protein production system to the outside of a host. The sequence of the secretory signal can be appropriately set depending on the type of the host.

[Nucleic acid]

The nucleic acid according to the present invention encodes the modified fibroin according to the present invention. Specific examples of the nucleic acid include nucleic acids encoding a modified fibroin including an amino acid sequence set forth in SEQ ID NO: 3, SEQ ID NO: 4 or SEQ ID NO: 10, or a protein having an amino acid sequence (tag sequence) set forth in SEQ ID NO: 5 attached to either or both of the N-terminal and C-terminal of these amino acid sequences, or the like.

The nucleic acid according to one embodiment is a nucleic acid which hybridizes with a complementary strand of a nucleic acid encoding the modified fibroin according to the present invention under stringent conditions and which encodes a modified fibroin including a domain sequence represented by Formula 1: $[(A)_n$ motif-REP$]_m$, in which z/w is 50.9% or more in the case where the total number of amino acid residues in the amino acid sequence consisting of XGX (where X represents an amino acid residue other than glycine) contained in REP is defined as z, and the total number of amino acid residues of REP in the domain sequence is defined as w.

The term "stringent conditions" refers to conditions under which a so-called specific hybrid is formed and a non-specific hybrid is not formed. The "stringent conditions" may be any of low stringent conditions, moderately stringent conditions and highly stringent conditions. The low stringent conditions mean that hybridization occurs only in the case where there is at least 85% or more identity between the sequences, and include, for example, conditions of hybridization at 42°C using 5×SSC containing 0.5% SDS. The moderately stringent conditions mean that hybridization occurs only in the case where there is at least 90% or more identity between the sequences, and include, for example, conditions of hybridization at 50°C using 5×SSC containing 0.5% SDS. The highly stringent conditions mean that hybridization occurs only in the case where there is at least 95% or more identity between the sequences, and include, for example, conditions of hybridization at 60°C using 5×SSC containing 0.5% SDS.

The nucleic acid according to one embodiment is a nucleic acid which has 90% or more sequence identity with a nucleic acid encoding the modified fibroin according to the present invention and which encodes a modified fibroin including a domain sequence represented by Formula 1: $[(A)_n$ motif-REP$]_m$, in which z/w is 50.9% or more in the case where the total number of amino acid residues in the amino acid sequence consisting of XGX (where X represents an amino acid residue other than glycine) contained in REP is defined as z, and the total number of amino acid residues of REP in the domain sequence is defined as w. It is preferred that the sequence identity is 95% or more.

[Host and expression vector]

An expression vector according to the present invention has a nucleic acid sequence according to the present invention and one or a plurality of regulatory sequences operably linked thereto. The regulatory sequence is a sequence (for example, a promoter, an enhancer, a ribosome binding sequence, or a transcription termination sequence) that controls the expression of a recombinant protein in a host, and can be appropriately selected depending on the type of the host. The type of the expression vector such as a plasmid vector, a viral vector, a cosmid vector, a fosmid vector, or an artificial chromosome vector can be appropriately selected depending on the type of the host.

The host according to the present invention is a host which has been transformed with the expression vector according to the present invention. Both prokaryotes and eukaryotes such as yeast, filamentous fungi, insect cells, animal cells, and plant cells can be suitably used as hosts.

As the expression vector, an expression vector which can autonomously replicate in a host cell or can be incorporated into a chromosome of a host and which contains a promoter at a position capable of transcribing the nucleic acid according to the present invention is suitably used.

In the case where a prokaryote such as a bacterium is used as a host, the expression vector according to the present invention is preferably a vector which is capable of autonomous replication in the prokaryote and at the same time includes a promoter, a ribosome binding sequence, a nucleic acid according to the present invention and a transcription termination sequence. A gene that controls a promoter may be included.

Examples of the prokaryote include microorganisms belonging to the genus *Escherichia, Brevibacillus, Serratia, Bacillus, Microbacterium, Brevibacterium, Corynebacterium* and *Pseudomonas.*

Examples of microorganisms belonging to the genus *Escherichia* include *Escherichia coli* BL21 (Novagen, Inc.), *Escherichia coli* BL21 (DE3) (Life Technologies Corporation), *Escherichia coli* BLR (DE3) (Merck KGaA), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No. 49, *Escherichia coli* Rosetta (DE3) (Novagen, Inc.), *Escherichia coli* TB1, *Escherichia coli* Tuner (Novagen, Inc.), *Escherichia coli* Tuner (DE3) (Novagen, Inc.), *Escherichia coli* W1485, *Escherichia coli* W3110 (ATCC 27325), *Escherichia coli* XL1-Blue, and *Escherichia coli* XL2-Blue.

Examples of microorganisms belonging to the genus Brevibacillus include Brevibacillus agri, Brevibacillus borstelensis, Brevibacillus centrosporus, Brevibacillus formosus, Brevibacillus invocatus, Brevibacillus laterosporus, Brevibacillus limnophilus, Brevibacillus parabrevis, Brevibacillus reuszeri, Brevibacillus thermoruber, Brevibacillus brevis 47 (FERM BP-1223), Brevibacillus brevis 47K (FERM BP-2308), Brevibacillus brevis 47-5 (FERM BP-1664), Brevibacillus brevis 47-5Q (JCM 8975), Brevibacillus choshinensis HPD31 (FERM BP-1087), Brevibacillus choshinensis HPD31-S (FERM BP-6623), Brevibacillus choshinensis HPD31-OK (FERM BP-4573), and Brevibacillus choshinensis SP3 strain (manufactured by Takara Bio, Inc.).

Examples of microorganisms belonging to the genus Serratia include Serratia liquefaciens ATCC 14460, Serratia entomophila, Serratia ficaria, Serratia fonticola, Serratia grimesii, Serratia proteamaculans, Serratia odorifera, Serratia plymuthica, and Serratia rubidaea.

Examples of microorganisms belonging to the genus *Bacillus* include *Bacillus subtilis* and *Bacillus amylolique-*

*faciens.*

Examples of microorganisms belonging to the genus *Microbacterium* include *Microbacterium ammoniaphilum* ATCC 15354.

Examples of microorganisms belonging to the genus Brevibacterium include Brevibacterium divaricatum (Corynebacterium glutamicum) ATCC 14020, Brevibacterium flavum (Corynebacterium glutamicum ATCC 14067) ATCC 13826, ATCC 14067, Brevibacterium immariophilum ATCC 14068, Brevibacterium lactofermentum (Corynebacterium glutamicum ATCC 13869) ATCC 13665, ATCC 13869, Brevibacterium roseum ATCC 13825, Brevibacterium saccharolyticum ATCC 14066, Brevibacterium tiogenitalis ATCC 19240, Brevibacterium album ATCC 15111, and Brevibacterium cerinum ATCC 15112.

Examples of microorganisms belonging to the genus *Corynebacterium* include *Corynebacterium ammoniagenes* ATCC 6871, ATCC 6872, *Corynebacterium glutamicum* ATCC 13032, *Corynebacterium glutamicum* ATCC 14067, *Corynebacterium acetoacidophilum* ATCC 13870, *Corynebacterium·acetoglutamicum* ATCC 15806, *Corynebacterium alkanolyticum* ATCC 21511, *Corynebacterium callunae* ATCC 15991, *Corynebacterium glutamicum* ATCC 13020, ATCC 13032, ATCC 13060, *Corynebacterium lilium* ATCC 15990, *Corynebacterium melassecola* ATCC 17965, *Corynebacterium thermoaminogenes* AJ12340 (FERM BP-1539), and *Corynebacterium herculis* ATCC 13868.

Examples of microorganisms belonging to the genus Pseudomonas include Pseudomonas putida, Pseudomonas fluorescens, Pseudomonas brassicacearum, Pseudomonas fulva, and Pseudomonas sp. D-0110.

As a method for introducing an expression vector into the foregoing host cell, any method can be used as long as it introduces DNA into the host cell. Examples thereof include a method using calcium ions [Proc. Natl. Acad. Sci. USA, 69, 2110 (1972)], a protoplast method (Japanese Unexamined Patent Publication No. S63-248394), or a method described in Gene, 17, 107 (1982) or Molecular & General Genetics, 168, 111 (1979).

Transformation of microorganisms belonging to the genus *Brevibacillus* can be carried out, for example, by the method of Takahashi et al. (J. Bacteriol., 1983, 156: 1130-1134), the method of Takagi et al. (Agric. Biol. Chem., 1989, 53: 3099-3100), or the method of Okamoto et al. (Biosci. Biotechnol. Biochem., 1997, 61: 202-203).

Examples of the vector into which the nucleic acid according to the present invention is introduced (hereinafter, simply referred to as "vector") include pBTrp2, pBTac1, and pBTac2 (all commercially available from Boehringer Mannheim GmbH), pKK233-2 (manufactured by Pharmacia Corporation), pSE280 (manufactured by Invitrogen Corporation), pGEMEX-1 (manufactured by Promega Corporation), pQE-8 (manufactured by QIAGEN Corporation), pKYP10 (Japanese Unexamined Patent Publication No. S58-110600), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene Corporation), pTrs30 (constructed from *Escherichia coli* JM109/pTrS30 (FERM BP-5407)], pTrs32 [constructed from *Escherichia coli* JM109/pTrS32 (FERM BP-5408)], pGHA2 [constructed from *Escherichia coli* IGHA2 (FERM B-400), Japanese Unexamined Patent Publication No. S60-221091], pTerm2 (US 4686191, US 4939094, US 5160735), pSupex, pUB110, pTP5, pC194, pEG400 [J. Bacteriol., 172, 2392 (1990)], pGEX (manufactured by Pharmacia Corporation), and pET systems (manufactured by Novagen, Inc.).

In the case where *Escherichia coli* is used as a host, pUC18, pBluescriptII, pSupex, pET22b, pCold, or the like can be mentioned as a suitable vector.

Specific examples of vectors suitable for microorganisms belonging to the genus *Brevibacillus* include pUB110 or pHY500 (Japanese Unexamined Patent Publication No. H2-31682), pNY700 (Japanese Unexamined Patent Publication No. H4-278091), pHY4831 (J. Bacteriol., 1987, 1239-1245), pNU200 (UDAKA Shigezou, Journal of the Agricultural Chemical Society of Japan, 1987, 61: 669-676), pNU100 (Appl. Microbiol. Biotechnol., 1989, 30: 75-80), pNU211 (J. Biochem., 1992, 112: 488-491), pNU211R2L5 (Japanese Unexamined Patent Publication No. H7-170984), pNH301 (Appl. Environ. Microbiol., 1992, 58: 525-531), pNH326, pNH400 (J. Bacteriol., 1995, 177: 745-749), and pHT210 (Japanese Unexamined Patent Publication No. H6-133782), pHT110R2L5 (Appl. Microbiol. Biotechnol., 1994, 42: 358-363), which are known as *Bacillus subtilis* vectors; and pNCO2 (Japanese Unexamined Patent Publication No. 2002-238569) which is a shuttle vector between *Escherichia coli* and a microorganism belonging to the genus *Brevibacillus.*

The promoter is not limited as long as it functions in a host cell. Examples thereof include promoters derived from *Escherichia coli* or phage such as a trp promoter (Ptrp), a lac promoter, a PL promoter, a PR promoter, and a T7 promoter. Also, promoters artificially designed and modified, such as a promoter (Ptrp×2) in which two Ptrp are connected in series, a tac promoter, a lacT7 promoter, and a let I promoter, can also be used.

It is preferable to use a plasmid in which the distance between the Shine-Dalgarno sequence, which is a ribosome binding sequence, and the initiation codon is adjusted to an appropriate distance (for example, 6 to 18 bases). In the expression vector according to the present invention, a transcription termination sequence is not always necessary for the expression of the nucleic acid according to the present invention, but it is preferable to arrange a transcription termination sequence immediately below a structural gene.

Examples of eukaryotic hosts include yeast, filamentous fungi (mold and the like), and insect cells.

Examples of the yeast include yeasts belonging to the genus *Saccharomyces, Schizosaccharomyces, Kluyveromyces, Trichosporon, Schwanniomyces, Pichia, Candida, Yarrowia, Hansenula,* and the like. More specific examples of the yeast include *Saccharomyces cerevisiae, Schizosaccharomyces pombe, Kluyveromyces lactis, Kluyveromyces marxianus, Trichosporon pullulans, Schwanniomyces alluvius, Schwanniomyces occidentalis, Candida utilis, Pichia pastoris, Pichia angusta, Pichia methanolica, Pichia polymorpha, Pichia stipitis, Yarrowia lipolytica,* and *Hansenula polymorpha.*

It is preferred that the expression vector in the case where yeast is used as a host cell usually include an origin of replication (in the case where amplification in a host is required), a selection marker for propagation of the vector in *Escherichia coli,* a promoter and a terminator for recombinant protein expression in yeast, and a selection marker for yeast.

In the case where the expression vector is a non-integrating vector, it is preferable to further include an autonomously replicating sequence (ARS). This makes it possible to improve the stability of the expression vectors in cells (Myers, A. M., et al. (1986) Gene 45: 299-310).

Examples of the vector in the case where yeast is used as a host include YEP13 (ATCC 37115), YEp24 (ATCC 37051), YCp50 (ATCC 37419), YIp, pHS19, pHS15, pA0804, pHIL3O1, pHIL-S1, pPIC9K, pPICZα, pGAPZα, and pPICZ B.

The promoter is not limited as long as it can be expressed in yeast. Examples of the promoter include a promoter of glycolytic genes such as hexose kinase, a PHO5 promoter, a PGK promoter, a GAP promoter, an ADH promoter, a gal 1 promoter, a gal 10 promoter, a heat shock polypeptide promoter, an MFα1 promoter, a CUP 1 promoter, a pGAP promoter, a pGCW14 promoter, an AOX1 promoter, and an MOX promoter.

As a method for introducing an expression vector into yeast, any method can be used as long as it introduces DNA into yeast. Examples thereof include an electroporation method (Methods Enzymol., 194, 182 (1990)), a spheroplast method (Proc. Natl. Acad. Sci., USA, 81, 4889 (1984)), a lithium acetate method (J. Bacteriol., 153, 163 (1983)), and a method described in Proc. Natl. Acad. Sci. USA, 75, 1929 (1978).

Examples of filamentous fungi include fungi belonging to the genus Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus, and Rhizomucor.

Specific examples of filamentous fungi include Acremonium alabamense, Acremonium cellulolyticus, Aspergillus aculeatus, Aspergillus awamori, Aspergillus oryzae, Aspergillus sake, Aspergillus sojae, Aspergillus tubigensis, Aspergillus niger, Aspergillus nidulans, Aspergillus parasiticus, Aspergillus ficuum, Aspergillus phoenicus, Aspergillus foetidus, Aspergillus flavus, Aspergillus fumigatus, Aspergillus japonicus, Trichoderma viride, Trichoderma harzianum, Trichoderma reseei, Chrysosporium lucknowense, Thermoascus, Sporotrichum, Sporotrichum cellulophilum, Talaromyces, Thielavia terrestris, Thielavia, Neurospora crassa, Fusarium oxysporum, Fusarium graminearum, Fusarium venenatum, Humicola insolens, Penicillium chrysogenum, Penicillium camemberti, Penicillium canescens, Penicillium emersonii, Penicillium funiculosum, Penicillium griseoroseum, Penicillium purpurogenum, Penicillium roqueforti, Myceliophthora thermophilum, Mucor ambiguus, Mucor circinelloides, Mucor fragilis, Mucor hiemalis, Mucor inaequisporus, Mucor oblongiellipticus, Mucor racemosus, Mucor recurvus, Mucor saturninus, Mucor subtilissmus, Ogataea polymorpha, Phanerochaete chrysosporium, Rhizomucor miehei, Rhizomucor pusillus, and Rhizopus arrhizus.

The promoter in the case where the host is a filamentous fungus may be any one of a gene related to a glycolytic system, a gene related to constitutive expression, an enzyme gene related to hydrolysis, and the like. Specific examples thereof include amyB, glaA, agdA, glaB, TEF1, xynF1 tannase gene, No. 8AN, gpdA, pgkA, enoA, melO, sodM, catA, and catB.

Introduction of the expression vector into filamentous fungi can be carried out by a conventionally known method. Examples thereof include the method of Cohen et al. (calcium chloride method) [Proc. Natl. Acad. Sci. USA, 69: 2110 (1972)], a protoplast method [Mol. Gen. Genet., 168:111 (1979)], a competent method [J. Mol. Biol., 56: 209 (1971)], and an electroporation method.

Insect cells include, for example, lepidopteran insect cells, more specifically insect cells derived from *Spodoptera frugiperda* such as Sf9 and Sf21, and insect cells derived from *Trichoplusia ni* such as High 5.

Examples of the vector in the case where an insect cell is used as a host include baculoviruses such as *Autographa californica* nuclear polyhedrosis virus which is a virus that infects insects belonging to the family *Noctuidae* (Baculovirus Expression Vectors, A Laboratory Manual, W. H. Freeman and Company, New York (1992)).

In the case where an insect cell is used as a host, a polypeptide can be expressed by the method described in, for example, Current Protocols in Molecular Biology, Baculovirus Expression Vectors, A Laboratory Manual, W. H. Freeman and Company, New York (1992), or Bio/Technology, 6, 47 (1988). That is, a recombinant gene transfer vector and a baculovirus are co-introduced into an insect cell to obtain a recombinant virus (expression vector) in an insect cell culture supernatant, and then the recombinant virus is further infected into an insect cell, whereby the polypeptide can be expressed. Examples of the gene transfer vector used in the above method include pVL1392, pVL1393, and pBlue-BacIII (all manufactured by Invitorogen Corporation).

As a method for co-introducing a recombinant gene transfer vector and a baculovirus into an insect cell for constructing the recombinant virus, for example, a calcium phosphate method (Japanese Unexamined Patent Publication No. H2-227075), a lipofection method (Proc. Natl. Acad. Sci. USA, 84, 7413 (1987)), or the like can be mentioned.

The recombinant vector according to the present invention preferably further contains a selection marker gene for selecting a transformant. For example, in *Escherichia coli,* resistance genes for various drugs such as tetracycline, ampicillin, and kanamycin can be used as selection marker genes. A recessive selection marker capable of complementing a genetic mutation involved in auxotrophy can also be used. In yeast, a resistance gene for geneticin can be used as a selection marker gene, and a gene complementing a genetic mutation involved in auxotrophy, or a selection marker such as LEU2, URA3, TRP1, or HIS3 can also be used. Examples of the selection marker gene for filamentous fungi include a marker gene selected from the group consisting of niaD (Biosci. Biotechnol. Biochem., 59, 1795-1797 (1995)), argB (Enzyme Microbiol Technol, 6, 386-389, (1984)), sC (Gene, 84, 329-334, (1989)), ptrA (BiosciBiotechnol Biochem, 64, 1416-1421, (2000)), pyrG (BiochemBiophys Res Commun, 112, 284-289, (1983)), amdS (Gene, 26, 205-221, (1983)), aureobasidin resistance gene (Mol Gen Genet, 261, 290-296, (1999)), benomyl resistance gene (Proc Natl Acad Sci USA, 83, 4869-4873, (1986)) and hygromycin resistance gene (Gene, 57, 21-26, (1987)), and a leucine auxotrophy-complementing gene. Further, in the case where the host is an auxotrophic mutant strain, a wild-type gene complementing the auxotrophy can also be used as a selection marker gene.

The selection of the host transformed with the expression vector according to the present invention can be carried out by plaque hybridization and colony hybridization using a probe that selectively binds to the nucleic acid according to the present invention. As the probe, it is possible to use a probe obtained by modifying a partial DNA fragment amplified by a PCR method based on sequence information of the nucleic acid according to the present invention with a radioisotope or digoxigenin.

(Production of modified fibroin)

In the host transformed with the expression vector according to the present invention, the modified fibroin according to the present invention can be produced by expressing the nucleic acid according to the present invention. As for the expression method, secretory production, fusion protein expression, or the like, in addition to direct expression, can be carried out according to the method described in Molecular Cloning, 2nd edition. In the case where it is expressed by yeast, an animal cell, or an insect cell, a modified fibroin can be obtained as a polypeptide to which a sugar or sugar chain is added.

The modified fibroin according to the present invention can be produced, for example, by culturing a host transformed with the expression vector according to the present invention in a culture medium, producing and accumulating the modified fibroin according to the present invention in the culture medium, and then collecting the modified fibroin from the culture medium. The method for culturing the host according to the present invention in a culture medium can be carried out according to a method commonly used for culturing a host.

In the case where the host according to the present invention is a prokaryote such as *Escherichia coli* or a eukaryote such as yeast, any of a natural medium and a synthetic medium may be used as a culture medium of the host according to the present invention as long as it contains a carbon source, a nitrogen source, inorganic salts and the like which can be assimilated by the host and it is capable of efficiently culturing the host.

As the carbon source, any carbon source that can be assimilated by the host may be used. Examples of the carbon source that can be used include carbohydrates such as glucose, fructose, sucrose, and molasses, starch and starch hydrolyzates containing them, organic acids such as acetic acid and propionic acid, and alcohols such as ethanol and propanol.

Examples of the nitrogen source that can be used include ammonium salts of inorganic or organic acids such as ammonia, ammonium chloride, ammonium sulfate, ammonium acetate and ammonium phosphate, other nitrogen-containing compounds, peptone, meat extract, yeast extract, corn steep liquor, casein hydrolyzate, soybean cake and soybean cake hydrolyzate, various fermented microbial cells and digested products thereof.

Examples of the inorganic salt that can be used include potassium dihydrogen phosphate, dipotassium phosphate, magnesium phosphate, magnesium sulfate, sodium chloride, ferrous sulfate, manganese sulfate, copper sulfate, and calcium carbonate.

Culture of a prokaryote such as *Escherichia coli* or a eukaryote such as yeast can be carried out under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium can be adjusted using an inorganic acid, an organic acid, an alkali solution, urea, calcium carbonate, ammonia, or the like.

In addition, antibiotics such as ampicillin and tetracycline may be added to the culture medium as necessary during the culture. In the case of culturing a microorganism transformed with an expression vector using an inducible promoter as a promoter, an inducer may be added to the medium as necessary. For example, in the case of culturing a microorganism transformed with an expression vector using a lac promoter, isopropyl-β-D-thiogalactopyranoside or the like is used, and in the case of culturing a microorganism transformed with an expression vector using a trp promoter, indole acrylic acid or the like may be added to the medium.

As a culture medium for insect cells, commonly used TNM-FH medium (manufactured by Pharmingen Inc.), Sf-900 II SFM medium (manufactured by Life Technologies Corporation), ExCell 400 and ExCell 405 (both manufactured by JRH Biosciences Inc.), Grace's Insect Medium (Nature, 195, 788 (1962)), and the like can be used.

Culture of insect cells can be carried out, for example, for a culture time of 1 to 5 days under conditions such as pH 6 to 7 of culture medium and culture temperature 25°C to 30°C. In addition, an antibiotic such as gentamicin may be added to the culture medium as necessary during the culture.

In the case where the host is a plant cell, the transformed plant cell may be directly cultured, or it may be differentiated into a plant organ and then cultured. As the culture medium for culturing a plant cell, for example, commonly used Murashige and Skoog (MS) medium, White medium, or a medium in which a plant hormone such as auxin or cytokinin is added to these media can be used.

Culture of animal cells can be carried out, for example, for a culture time of 3 to 60 days under conditions such as pH 5 to 9 of the culture medium and culture temperature 20°C to 40°C. In addition, an antibiotic such as kanamycin or hygromycin may be added to the medium as necessary during the culture.

As a method for producing a modified fibroin using a host transformed with the expression vector according to the present invention, there are a method for producing the modified fibroin in a host cell, a method for secreting the modified fibroin outside the host cell, and a method for producing the modified fibroin on the outer membrane of the host cell. Each of these methods can be selected by changing the host cell to be used and the structure of the modified fibroin to be produced.

For example, in the case where a modified fibroin is produced in the host cell or on the outer membrane of the host cell, the production method can be altered to actively secrete the modified fibroin outside the host cell according to the method of Paulson et al. (J. Biol. Chem., 264, 17619 (1989)), the method of Lowe et al. (Proc. Natl. Acad. Sci. USA, 86, 8227 (1989), Genes Develop., 4, 1288 (1990)), or the methods described in Japanese Unexamined Patent Publication No. H5-336963, International Publication No. WO94/23021, and the like. That is, the modified fibroin can be actively secreted outside the host cell by expressing the modified fibroin in a form in which a signal peptide is added to a polypeptide containing an active site of a modified fibroin using a gene recombination technique.

The modified fibroin produced by the host transformed with the expression vector according to the present invention can be isolated and purified by a method commonly used for protein isolation and purification. For example, in the case where the modified fibroin is expressed in a dissolved state in cells, the host cells are recovered by centrifugation after completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation can be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (Pharmacia Corporation), a hydrophobic chromatography method using a resin such as butyl sepharose or phenyl sepharose, a gel filtration method using a molecular sieve, an affinity chromatography method, a chromatofocusing method, an electrophoresis method such as isoelectric focusing or the like, alone or in combination thereof.

As the chromatography, column chromatography using phenyl-TOYOPEARL (available from Tosoh Corporation), DEAE-TOYOPEARL (available from Tosoh Corporation), and Sephadex G-150 (available from Pharmacia Biotech Inc.) is preferably used.

In the case where the modified fibroin is expressed by the formation of an insoluble matter in the cell, similarly, the host cells are recovered, disrupted and centrifuged to recover the insoluble matter of the modified fibroin as a precipitated fraction. The recovered insoluble matter of the modified fibroin can be solubilized with a protein denaturing agent. After this operation, a purified preparation of modified fibroin can be obtained by the same isolation and purification method as described above.

In the case where a modified fibroin or a derivative in which a sugar chain has been added to the modified fibroin is secreted extracellularly, the modified fibroin or the derivative thereof can be recovered from the culture supernatant. That is, a culture supernatant is obtained by treating the culture by a technique such as centrifugation, and a purified preparation can be obtained from the culture supernatant by using the same isolation and purification method as described above.

(Spinning)

The modified fibroin according to the present invention may be further subjected to spinning after production and purification as described above. The modified fibroin according to the present invention can be spun by a method commonly used for spinning fibroin. For example, a fiber formed from the modified fibroin according to the present invention can be obtained by spinning a spinning solution (dope solution) in which the modified fibroin according to the present invention is dissolved in a solvent.

The spinning solution is prepared by adding a solvent to the modified fibroin and adjusting it to a spinnable viscosity. The solvent may be any solvent as long as it can dissolve the modified fibroin. Examples of the solvent include hexafluoroisopropanol (HFIP), hexafluoroacetone (HFA), dimethyl sulfoxide (DMSO), N,N-dimethylformamide (DMF), formic acid, aqueous solutions containing urea, guanidine, sodium dodecyl sulfate (SDS), lithium bromide, calcium chloride, and lithium thiocyanate.

An inorganic salt may be added to the spinning solution, as necessary. The inorganic salt may be, for example, an inorganic salt consisting of a Lewis acid and a Lewis base shown below. Examples of the Lewis base include an oxo acid ion (nitrate ion, perchlorate ion, or the like), a metal oxo acid ion (permanganate ion or the like), a halide ion, a thiocyanate ion, and a cyanate ion. Examples of the Lewis acid include a metal ion such as an alkali metal ion or an alkaline earth metal ion, a polyatomic ion such as an ammonium ion, and a complex ion. Specific examples of the inorganic salt consisting of a Lewis acid and a Lewis base include lithium salts such as lithium chloride, lithium bromide, lithium iodide, lithium nitrate, lithium perchlorate, and lithium thiocyanate; calcium salts such as calcium chloride, calcium bromide, calcium iodide, calcium nitrate, calcium perchlorate, and calcium thiocyanate; iron salts such as iron chloride, iron bromide, iron iodide, iron nitrate, iron perchlorate, and iron thiocyanate; aluminum salts such as aluminum chloride, aluminum bromide, aluminum iodide, aluminum nitrate, aluminum perchlorate, and aluminum thiocyanate; potassium salts such as potassium chloride, potassium bromide, potassium iodide, potassium nitrate, potassium perchlorate, and potassium thiocyanate; sodium salts such as sodium chloride, sodium bromide, sodium iodide, sodium nitrate, sodium perchlorate, and sodium thiocyanate; zinc salts such as zinc chloride, zinc bromide, zinc iodide, zinc nitrate, zinc perchlorate, and zinc thiocyanate; magnesium salts such as magnesium chloride, magnesium bromide, magnesium iodide, magnesium nitrate, magnesium perchlorate, and magnesium thiocyanate; barium salts such as barium chloride, barium bromide, barium iodide, barium nitrate, barium perchlorate, and barium thiocyanate; and strontium salts such as strontium chloride, strontium bromide, strontium iodide, strontium nitrate, strontium perchlorate, and strontium thiocyanate.

The viscosity of the spinning solution may be appropriately set according to the spinning method, and it can be set to 100 to 15,000 centipoise (cP) at 35°C, for example. The viscosity of the spinning solution can be measured, for example, by using an "EMS viscometer" (trade name, manufactured by Kyoto Electronics Manufacturing Co., Ltd.).

The spinning method is not particularly limited as long as it is a method capable of spinning the modified fibroin according to the present invention, and examples thereof include dry spinning, melt spinning, and wet spinning. As a preferred spinning method, wet spinning can be mentioned.

In wet spinning, a solvent in which a modified fibroin is dissolved is extruded from a spinneret (nozzle) into a coagulation liquid (coagulation liquid tank), and the modified fibroin is solidified in the coagulation liquid, whereby it is possible to obtain an undrawn yarn in the form of a thread. The coagulation liquid may be a solution capable of desolvation, and examples thereof include lower alcohols having 1 to 5 carbon atoms such as methanol, ethanol and 2-propanol, and acetone. Water may be appropriately added to the coagulation liquid. The temperature of the coagulation liquid is preferably 0°C to 30°C. In the case where a syringe pump having a nozzle with a diameter of 0.1 to 0.6 mm is used as a spinneret, the extrusion rate is preferably 0.2 to 6.0 ml/hr and more preferably 1.4 to 4.0 ml/hr per hole. The length of the coagulation liquid tank may be any length as long as desolvation can be carried out efficiently, and it is, for example, 200 to 500 mm. The take-off speed of the undrawn yarn may be, for example, 1 to 20 m/min and preferably 1 to 3 m/min. The residence time may be, for example, 0.01 to 3 minutes and preferably 0.05 to 0.15 minutes. Further, drawing (pre-drawing) may be carried out in the coagulation liquid. In order to suppress the evaporation of a lower alcohol, the coagulation liquid may be maintained at a low temperature and the yarn may be taken off in the state of an undrawn yarn. The coagulation liquid tank may be provided in multiple stages, and the drawing may be carried out in each stage or a specific stage, as necessary.

The undrawn yarn (or pre-drawn yarn) obtained by the above method can be made into a drawn yarn (fibroin fiber) through a drawing step. As a drawing method, wet heat drawing, dry heat drawing, and the like can be mentioned.

The wet heat drawing can be carried out in warm water, in a solution obtained by adding an organic solvent or the like to warm water, or during steam heating. The temperature may be, for example, 50°C to 90°C and preferably 75°C to 85°C. In wet heat drawing, undrawn yarn (or pre-drawn yarn) can be drawn, for example, 1 to 10 times, preferably 2 to 8 times.

Dry heat drawing can be carried out using an electric tube furnace, a dry heat plate, or the like. The temperature may be, for example, 140°C to 270°C and preferably 160°C to 230°C. In dry heat drawing, undrawn yarn (or pre-drawn yarn) can be drawn, for example, 0.5 to 8 times, preferably 1 to 4 times.

The wet heat drawing and the dry heat drawing may be carried out individually, or they may be carried out in multiple stages or in combination. That is, wet heat drawing and dry heat drawing can be carried out in an appropriate combination in such a manner that the first stage drawing is carried out by wet heat drawing and the second stage drawing is carried out by dry heat drawing, or the first stage drawing is carried out by wet heat drawing and the second stage drawing is carried out by wet heat drawing, and the third stage drawing is further carried out by dry heat drawing.

The final draw ratio in the drawing step is, for example, 5 to 20 times and preferably 6 to 11 times, with respect to the undrawn yarn (or pre-drawn yarn).

The modified fibroin according to the present invention may be chemically crosslinked between polypeptide molecules in a fibroin fiber after being drawn into the fibroin fiber. Examples of the functional group which can be crosslinked include an amino group, a carboxyl group, a thiol group, and a hydroxy group. For example, an amino group of a lysine side chain contained in a polypeptide can be crosslinked with a carboxyl group of a glutamic acid or aspartic acid side chain by an amide bond through dehydration condensation. The crosslinking may be carried out by a dehydration condensation reaction under vacuum heating or may be carried out by using a dehydrating condensation agent such as carbodiimide.

The crosslinking between polypeptide molecules may be carried out using a crosslinking agent such as carbodiimide or glutaraldehyde or may be carried out using an enzyme such as transglutaminase. The carbodiimide is a compound represented by General Formula: $R_1N=C=NR_2$ (where $R_1$ and $R_2$ each independently represent an organic group including an alkyl group or cycloalkyl group having 1 to 6 carbon atoms). Specific examples of the carbodiimide include 1-ethyl-3-(3-dimethylaminopropyl)carbodiimide hydrochloride (EDC), N,N'-dicyclohexylcarbodiimide (DCC), 1-cyclohexyl-3-(2-morpholinoethyl)carbodiimide, and diisopropyl carbodiimide (DIC). Among them, EDC and DIC are preferred since they have a high ability to form an amide bond between polypeptide molecules and facilitate a crosslinking reaction.

The crosslinking treatment is preferably carried out by applying a crosslinking agent to the fibroin fiber and crosslinking it by vacuum heating drying. As the crosslinking agent, a pure product may be applied to the fibroin fiber. Alternatively, the crosslinking agent may be added to the fibroin fiber by diluting a pure product with a lower alcohol having 1 to 5 carbon atoms and a buffer solution or the like to a concentration of 0.005 to 10% by mass. The crosslinking treatment is preferably carried out at a temperature of 20°C to 45°C for 3 to 42 hours. By the crosslinking treatment, higher stress (strength) can be imparted to the fibroin fiber.

[Product]

The fibroin fiber formed from the modified fibroin according to the present invention can be applied as a fiber or a yarn to a woven fabric, a knitted fabric, a combination thereof, a nonwoven fabric, or the like. Such a fibroin fiber can also be applied to high strength applications such as ropes, surgical sutures, flexible stops for electrical parts, and physiologically active materials for implantation (for example, artificial ligament and aortic band).

The modified fibroin according to the present invention can also be applied to filaments, films, foams, spheres, nanofibrils, hydrogels, resins and equivalents thereof, which can be produced in accordance with the method described in Japanese Unexamined Patent Publication No. 2009-505668, Japanese Unexamined Patent Publication No. 2009-505668, Japanese Patent No. 5678283, Japanese Patent No. 4638735, or the like.

**Examples**

Hereinafter, the present invention will be described more specifically with respect to Examples. However, the present invention is not limited to the following Examples.

[(1) Synthesis of nucleic acid encoding modified fibroin and construction of expression vector]

Based on the nucleotide sequence and amino acid sequence of *Nephila clavipes* (GenBank Accession Number: P46804.1, GI: 1174415) which is naturally occurring fibroin, fibroins and modified fibroins having amino acid sequences set forth in SEQ ID NOs: 1 to 4 and 6 to 11 were designed. The amino acid sequence set forth in SEQ ID NO: 1 (Met-PRT313) is a sequence obtained by deleting alanine residues of an amino acid sequence in which the alanine residues in the $(A)_n$ motif of the naturally occurring fibroin are consecutive so that the number of consecutive alanine residues is 5; and the amino acid sequence (PRT313) set forth in SEQ ID NO: 6 is a sequence obtained by adding the amino acid sequence (tag sequence and hinge sequence) set forth in SEQ ID NO: 5 to the N-terminal of the amino acid sequence set forth in SEQ ID NO: 1 (Comparative Examples 1 and 2). The amino acid sequence (Met-PRT399) set forth in SEQ ID NO: 2 is a sequence obtained by deleting the $(A)_n$ motif $((A)_5)$ every other two positions from the N-terminal side to the C-terminal side from the amino acid sequence set forth in SEQ ID NO: 1, and inserting one [$(A)_n$ motif-REP] before the C-terminal sequence; and the amino acid sequence (PRT399) set forth in SEQ ID NO: 7 is a sequence obtained by adding the amino acid sequence (tag sequence and hinge sequence) set forth in SEQ ID NO: 5 to the N-terminal of the amino acid sequence set forth in SEQ ID NO: 2 (Reference Examples 1 and 2). The amino acid sequence (Met-PRT380) set forth in SEQ ID NO: 3 is a sequence obtained by substituting GQX for all GGX in REP of the amino acid sequence set forth in SEQ ID NO: 1; and the amino acid sequence (PRT380) set forth in SEQ ID NO: 8 is a sequence obtained by adding the amino acid sequence (tag sequence and hinge sequence) set forth in SEQ ID NO: 5 to the N-terminal of the amino acid sequence set forth in SEQ ID NO: 3 (Examples 1 and 4). The amino acid sequence (Met-PRT410) set forth in SEQ ID NO: 4 is a sequence obtained by substituting GQX for all GGX in REP of the amino acid sequence set forth in SEQ ID NO: 2; and the amino acid sequence (PRT410) set forth in SEQ ID NO: 9 is a sequence obtained by adding the amino acid sequence (tag sequence and hinge sequence) set forth in SEQ ID NO: 5 to the N-terminal of the amino acid sequence set forth in SEQ ID NO: 4 (Examples 2 and 5). The amino acid sequence (Met-PRT468) set forth in SEQ ID NO: 10 is a sequence obtained by inserting two alanine residues at the C-terminal side of each $(A)_n$ motif of the amino acid sequence set forth in SEQ ID NO: 4 and further substituting a part of glutamine (Q) residues with a serine (S) residue to delete a part of amino acids on the N-terminal side so as to be almost the same as the molecular weight of SEQ ID NO: 4; and the amino acid sequence (PRT468) set forth in SEQ ID NO: 11 is a sequence obtained by adding the amino acid sequence (tag sequence and hinge sequence) set forth in SEQ ID NO: 5 to the N-terminal of the amino acid sequence set forth in SEQ ID NO: 10 (Example 3).

Each of nucleic acids encoding proteins having amino acid sequences set forth in SEQ ID NOs: 6 to 9 and 11 in which a His tag sequence and a hinge sequence (SEQ ID NO: 5) have been added to the N-terminal of each designed amino acid sequence set forth in SEQ ID NOs: 1 to 4 and 10 was synthesized. In the nucleic acid, an *Nde*I site was added to the 5' end and an *EcoR*I site was added downstream of the stop codon. These four kinds of nucleic acids were cloned into a cloning vector (pUC118). Thereafter, the same nucleic acid was cleaved by restriction enzyme treatment with *Nde*I and *EcoR*I, and then recombined into a protein expression vector pET-22b(+) to obtain an expression vector.

[(2) Expression of protein]

*Escherichia coli* BLR (DE3) was transformed with a pET22b(+) expression vector including each of nucleic acids encoding proteins having the amino acid sequences set forth in SEQ ID NOs: 6 to 9 and 11. The transformed *Escherichia coli* was cultured in 2 mL of an LB medium containing ampicillin for 15 hours. The culture solution was added to 100 mL of a seed culture medium (Table 6) containing ampicillin so that the $OD_{600}$ was 0.005. The temperature of the culture solution was maintained at 30°C and the flask culture was carried out (for about 15 hours) until the $OD_{600}$ reached 5, thereby obtaining a seed culture solution.

[Table 6] Seed culture medium

| Reagents | Concentration (g/L) |
|---|---|
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter to which 500 ml of a production medium (Table 7) had been added so that the $OD_{600}$ was 0.05. The culture was carried out while maintaining the culture solution temperature at 37°C and keeping the pH constant at 6.9. Further, the dissolved oxygen concentration in the culture solution was maintained at 20% of the dissolved oxygen saturation concentration.

[Table 7] Production medium

| Reagents | Concentration (g/L) |
|---|---|
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADEKANOL (LG-295S, Adeka Corporation) | 0.1 (mL/L) |

Immediately after glucose in the production medium was completely consumed, a feed solution (455 g/1L of glucose and 120 g/1L of Yeast Extract) was added at a rate of 1 ml/min. The culture was carried out while maintaining the culture solution temperature at 37°C and keeping the pH constant at 6.9. Further, the dissolved oxygen concentration in the culture solution was maintained at 20% of the dissolved oxygen saturation concentration, and the culture was carried out for 20 hours. Thereafter, 1 M isopropyl-β-thiogalactopyranoside (IPTG) was added to the culture solution to a final concentration of 1 mM to induce the expression of the target protein. Twenty hours after addition of IPTG, the culture solution was centrifuged to recover the bacterial cells. SDS-PAGE was carried out using the bacterial cells prepared from the culture solution before the addition of IPTG and after the addition of IPTG, and the expression of the target protein was confirmed by the appearance of a band of a target protein size depending on the addition of IPTG.

[(3) Purification of protein]

The bacterial cells recovered 2 hours after the addition of IPTG were washed with 20 mM Tris-HCl buffer solution (pH 7.4). The bacterial cells after washing were suspended in 20 mM Tris-HCl buffer solution (pH 7.4) containing about 1 mM PMSF, and the cells were disrupted with a high-pressure homogenizer (available from GEA Niro Soavi SpA). The disrupted cells were centrifuged to obtain a precipitate. The obtained precipitate was washed with 20 mM Tris-HCl buffer solution (pH 7.4) until high purity. The precipitate after washing was suspended in 8 M guanidine buffer solution (8 M guanidine hydrochloride, 10 mM sodium dihydrogen phosphate, 20 mM NaCl, 1 mM Tris-HCl, pH 7.0) so as to have a concentration of 100 mg/mL, and dissolved by stirring with a stirrer at 60°C for 30 minutes. After dissolution, dialysis was carried out with water using a dialysis tube (cellulose tube 36/32 manufactured by Sanko Junyaku Co., Ltd.). The white aggregated protein obtained after dialysis was recovered by centrifugation, the water content was removed with a freeze dryer, and the freeze-dried powder was recovered.

The degree of purification of the target protein in the freeze-dried powder thus obtained was confirmed by image analysis of polyacrylamide gel electrophoresis results of the powder using TotalLab (Nonlinear Dynamics Ltd.). As a result, the purity of each protein was about 85%.

[(4) Preparation of spinning solution (dope solution)]

Using DMSO in which 4% by mass of lithium chloride as an additive was previously dissolved as a main solvent, each freeze-dried powder of PRT313 (SEQ ID NO: 6: Comparative Example 1), PRT399 (SEQ ID NO: 7: Reference Example 1), PRT380 (SEQ ID NO: 8: Example 1), PRT410 (SEQ ID NO: 9: Example 2) and PRT468 (SEQ ID NO: 11: Example 3) proteins as prepared above was added to the main solvent to a concentration of 24% by mass. The freeze-dried powder was dissolved in a rotator at 90° for 1 hour and at 80°C for 15 hours and then filtered through a sintered metal filter to remove dust. Subsequently, the filtrate was allowed to stand for 1 hour to remove foam to thereby prepare a spinning solution (dope solution). Although the viscosity of the spinning solution varies somewhat depending on the protein type and temperature, in the case of PRT410, it was 5,000 cP (centipoise) at 35°C.

[(5) Spinning]

The spinning solution was filled in a reserve tank and discharged from a multihole nozzle having a diameter of 0.1 or 0.2 mm into a 100% by mass methanol coagulation bath using a gear pump. The discharge amount was adjusted to 3 to 6 ml/min. After coagulation, washing and drawing were carried out in a 100% by mass methanol washing bath. After washing and drawing, it was dried using a dry hot plate and the obtained original yarn (fiber) was wound up.

[Measurement of physical properties]

Physical properties of the obtained original yarn were measured as follows.

(A) Fiber diameter was determined using an optical microscope.
(B) The stress, initial elastic modulus, and elongation (displacement at breakage, displacement) of the fiber were measured at a temperature of 20°C and a relative humidity of 65% using a tensile tester (INSTRON 3342), and the toughness was calculated by the following formula. In the tensile test, it was measured at intervals of 10 ms. Each sample was adhered to a mold made of cardboard, the distance between the clamps was 20 mm, and the pulling speed was 10 mm/min. The load cell capacity was 10 N, and the clamping jig was clip type. The measured value was the average value of the number of samples n = 5.

Toughness was calculated by the following calculation formula.

$$\text{Toughness} = [E/(r^2 \times \pi \times L) \times 1000] \ (\text{unit: MJ/m}^3)$$

in which

E: Fracture energy (unit: J)
r: Radius of fiber (unit: mm)
$\pi$: Pi
L: Distance between the clamps at the time of tensile test measurement: 20 mm

The amount of production of the frozen powder of each protein, and the stress, toughness and elongation of each original yarn were measured, and the results are shown in Table 8 as relative values in the case where the value of PRT313 (SEQ ID NO: 6: Comparative Example 1) is 100.

[Table 8]

| | Designation | Amount of production of powder (%) | Stress (%) | Toughness (%) | Elongation (%) |
|---|---|---|---|---|---|
| Comparative Example 1 | PRT313 | 100 | 100 | 100 | 100 |
| Reference Example 1 | PRT399 | 297 | - | - | - |
| Example 1 | PRT380 | 469 | - | - | - |
| Example 2 | PRT410 | 579 | 84 | 108 | 131 |
| Example 3 | PRT468 | 762 | 69 | 113 | 164 |

Modified fibroin with a reduced content of glycine residues in REP exhibited significantly improved productivity (Example 1). The modified fibroin with a reduced content of $(A)_n$ motif, in addition to having a reduced content of glycine residues in REP, exhibited more significantly improved productivity and improved toughness and elongation (Examples 2 and 3).

Next, the spinning conditions were changed as shown below, and the purified proteins PRT313 (SEQ ID NO: 6: Comparative Example 2), PRT399 (SEQ ID NO: 7: Reference Example 2), PRT380 (SEQ ID NO: 8: Example 4) and PRT410 (SEQ ID NO: 9: Example 5) as prepared above were subjected to spinning. Physical properties of the proteins were measured and compared in the same manner as described above.

The spinning solution was prepared in the same manner as in the foregoing section "(4) Preparation of spinning solution (dope solution)". The prepared spinning solution was filled in a reserve tank and discharged from a nozzle with a diameter of 0.2 mm into a 100% by mass methanol coagulation bath using a gear pump. The discharge amount was adjusted to 0.050 to 0.052 ml/min. After coagulation, washing was carried out in a 100% by mass methanol washing bath, and 3-fold drawing was carried out in a hot water bath at 50°C. After washing and drawing, it was dried using a hot roller at 60°C, and the obtained original yarn (fiber) was wound up.

The measurement results of the stress, toughness and elongation of each original yarn are shown in Table 9 as relative values in the case where the value of PRT313 (SEQ ID NO: 6: Comparative Example 2) is 100.

[Table 9]

| | Designation | Stress (%) | Toughness (%) | Elongation (%) |
|---|---|---|---|---|
| Comparative Example 2 | PRT313 | 100.0 | 100.0 | 100.0 |
| Reference Example 2 | PRT399 | 102.5 | 131.5 | 152.8 |
| Example 4 | PRT380 | 99.8 | 89.0 | 94.4 |
| Example 5 | PRT410 | 92.2 | 125.1 | 168.4 |

Modified fibroin with a reduced content of glycine residues in REP maintained strength (stress and toughness) and elongation (Example 4). Modified fibroin with a reduced $(A)_n$ motif content, in addition to having a reduced content of glycine residues in REP, exhibited improved toughness and elongation while maintaining stress (Example 5).

## Claims

1. A modified fibroin, comprising:

a domain sequence represented by Formula 1: $[(A)_n$ motif-REP$]_m$,
wherein the domain sequence has an amino acid sequence having a reduced content of glycine residues equivalent to an amino acid sequence in which, at least, one or a plurality of the glycine residues in REP is substituted with another amino acid residue, as compared to naturally occurring fibroin.

[In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

2. The modified fibroin according to claim 1, wherein the domain sequence has an amino acid sequence equivalent to an amino acid sequence in which, at least, in at least one motif sequence selected from GGX and GPGXX (where X represents an amino acid residue other than glycine) in REP, one glycine residue in one or a plurality of the motif sequences is substituted with another amino acid residue, as compared to the naturally occurring fibroin.

3. The modified fibroin according to claim 2, wherein the ratio of the motif sequence having the substitution of a glycine residue with another amino acid residue is 10% or more with respect to the entire motif sequence.

4. A modified fibroin, comprising:

   a domain sequence represented by Formula 1: $[(A)_n \text{ motif-REP}]_m$,
   wherein z/w is 50.9% or more in the case where the total number of amino acid residues in the amino acid sequence consisting of XGX (where X represents an amino acid residue other than glycine) contained in all REPs in the sequence excluding the sequence from the $(A)_n$ motif located at the most C-terminal side to the C-terminal of the domain sequence from the domain sequence is defined as z, and the total number of amino acid residues in the sequence excluding the sequence from the $(A)_n$ motif located at the most C-terminal side to the C-terminal of the domain sequence from the domain sequence is defined as w.

   [In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

5. The modified fibroin according to any one of claims 1 to 4, wherein the fibroin has, in addition to an amino acid sequence corresponding to substitution of one or a plurality of glycine residues in REP with another amino acid residue, an amino acid sequence corresponding to substitution, deletion, insertion and/or addition of one or a plurality of amino acid residues, as compared to naturally occurring fibroin.

6. The modified fibroin according to claim 5, wherein the naturally occurring fibroin is a fibroin derived from an insect or a spider.

7. The modified fibroin according to claim 5, wherein the naturally occurring fibroin is a major ampullate spider protein (MaSp) or minor ampullate spider protein (MiSp) of spiders.

8. The modified fibroin according to any one of claims 5 to 7, wherein the domain sequence has an amino acid sequence equivalent to an amino acid sequence in which, at least, in at least one motif sequence selected from GGX and GPGXX (where X represents an amino acid residue other than glycine) in REP, one glycine residue in one or a plurality of the motif sequences is substituted with another amino acid residue, as compared to the naturally occurring fibroin, and wherein the ratio of the motif sequence having the substitution of a glycine residue with another amino acid residue is 10% or more with respect to the entire motif sequence.

9. The modified fibroin according to claim 8, wherein the another amino acid residue is an amino acid residue selected from the group consisting of a glutamine (Q) residue, a valine (V) residue, a leucine (L) residue, an isoleucine (I) residue, a methionine (M) residue, a proline (P) residue, a phenylalanine (F) residue, a tryptophan (W) residue, an asparagine (N) residue, a serine (S) residue, a lysine (K) residue and a glutamic acid (E) residue.

10. The modified fibroin according to claim 8, wherein the another amino acid residue is a glutamine (Q) residue.

11. The modified fibroin according to any one of claims 1 to 10, wherein the domain sequence further has an amino acid sequence having a reduced content of $(A)_n$ motif equivalent to an amino acid sequence in which, at least, one or a plurality of the $(A)_n$ motifs is deleted, as compared to the naturally occurring fibroin.

12. The modified fibroin according to claim 11, wherein the domain sequence has an amino acid sequence equivalent to an amino acid sequence in which, at least, one $(A)_n$ motif per one to three $(A)_n$ motifs from an N-terminal side to the C-terminal side is deleted, as compared to the naturally occurring fibroin.

13. The modified fibroin according to claim 11, wherein the domain sequence has an amino acid sequence equivalent to an amino acid sequence in which, at least, two consecutive $(A)_n$ motif deletions and one $(A)_n$ motif deletion are repeated in this order from an N-terminal side to the C-terminal side, as compared to the naturally occurring fibroin.

14. The modified fibroin according to any one of claims 11 to 13, wherein a maximum value of x/y is 20% or more, in the case where the number of amino acid residues in REPs of two adjacent $[(A)_n$ motif-REP] units is sequentially compared from the N-terminal side to the C-terminal side, and the number of amino acid residues in REP having a smaller number of amino acid residues is defined as 1, the total value of the number of amino acid residues in the two adjacent $[(A)_n$ motif-REP] units where the ratio of the number of amino acid residues in the other REP is 2 to 3.5 is defined as x, and the total number of amino acid residues of the domain sequence is defined as y.

15. A modified fibroin, comprising an amino acid sequence set forth in SEQ ID NO: 3, SEQ ID NO: 4 or SEQ ID NO: 10, or an amino acid sequence having 90% or more sequence identity with the amino acid sequence set forth in SEQ ID NO: 3, SEQ ID NO: 4 or SEQ ID NO: 10.

16. The modified fibroin according to any one of claims 1 to 15, further comprising a tag sequence at either or both of the N-terminal and the C-terminal.

17. The modified fibroin according to claim 16, wherein the tag sequence includes an amino acid sequence set forth in SEQ ID NO: 5.

18. A modified fibroin, comprising an amino acid sequence set forth in SEQ ID NO: 8, SEQ ID NO: 9 or SEQ ID NO: 11, or an amino acid sequence having 90% or more sequence identity with the amino acid sequence set forth in SEQ ID NO: 8, SEQ ID NO: 9 or SEQ ID NO: 11.

19. A nucleic acid encoding the modified fibroin according to any one of claims 1 to 18.

20. A nucleic acid that hybridizes with a complementary strand of the nucleic acid according to claim 19 under stringent conditions and encodes a modified fibroin including a domain sequence represented by Formula 1: $[(A)_n$ motif-REP]$_m$. [In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

21. A nucleic acid having 90% or more sequence identity with the nucleic acid according to claim 19 and encoding a modified fibroin including a domain sequence represented by Formula 1: $[(A)_n$ motif-REP]$_m$. [In Formula 1, $(A)_n$ motif represents an amino acid sequence consisting of 4 to 20 amino acid residues and the number of alanine residues relative to the total number of amino acid residues in the $(A)_n$ motif is 83% or more, REP represents an amino acid sequence consisting of 10 to 200 amino acid residues, m represents an integer of 8 to 300, a plurality of $(A)_n$ motifs may be the same amino acid sequence or different amino acid sequences, and a plurality of REPs may be the same amino acid sequence or different amino acid sequences.]

22. An expression vector, comprising the nucleic acid sequence according to any one of claims 19 to 21 and one or a plurality of regulatory sequences operably linked thereto.

23. The expression vector according to claim 22, which is a plasmid vector or a viral vector.

24. A host transformed with the expression vector according to claim 22 or 23.

25. The host according to claim 24, which is a prokaryote.

26. The host according to claim 25, wherein the prokaryote is a microorganism belonging to a genus selected from the group consisting of *Escherichia, Brevibacillus, Serratia, Bacillus, Microbacterium, Brevibacterium, Corynebacterium* and *Pseudomonas.*

27. The host according to claim 24, which is a eukaryote.

28. The host according to claim 27, wherein the eukaryote is a yeast, a filamentous fungus or an insect cell.

29. The host according to claim 28, wherein the yeast is a yeast belonging to a genus selected from the group consisting of *Saccharomyces, Schizosaccharomyces, Kluyveromyces, Trichosporon, Schwanniomyces, Pichia, Candida, Yarrowia* and *Hansenula.*

30. The host according to claim 29, wherein the yeast belonging to the genus *Saccharomyces* is *Saccharomyces cerevisiae,* the yeast belonging to the genus *Schizosaccharomyces* is *Schizosaccharomyces pombe,* and the yeast belonging to the genus *Kluyveromyces* is *Kluyveromyces lactis,* the yeast belonging to the genus *Trichosporon* is *Trichosporon pullulans,* the yeast belonging to the genus *Schwaniomyces* is *Schwanniomyces alluvius,* the yeast belonging to the genus *Pichia* is *Pichia pastoris,* the yeast belonging to the genus *Candida* is *Candida albicans,* the yeast belonging to the genus *Yarrowia* is *Yarrowia lipolytica,* and the yeast belonging to the genus *Hansenula* is *Hansenula polymorpha.*

31. The host according to claim 28, wherein the filamentous fungus is a filamentous fungus belonging to a genus selected from the group consisting of *Aspergillus, Penicillium* and *Mucor.*

32. The host according to claim 31, wherein the filamentous fungus belonging to the genus *Aspergillus* is *Aspergillus oryzae,* the filamentous fungus belonging to the genus *Penicillium* is *Penicillium chrysogenum,* and the filamentous fungus belonging to the genus *Mucor* is *Mucor fragilis.*

33. The host according to claim 28, wherein the insect cell is a lepidopteran insect cell.

34. The host according to claim 28, wherein the insect cell is an insect cell derived from *Spodoptera frugiperda* or an insect cell derived from *Trichoplusia ni.*

35. A product comprising the modified fibroin according to any one of claims 1 to 18 and selected from the group consisting of a fiber, a yarn, a filament, a film, a foam, a sphere, a nanofibril, a hydrogel, a resin and an equivalent thereof.

Fig.1

Fig.2

50AA    100AA    10AA    20AA    30AA

AAAA    AAAA    AAAA    AAAA    AAAA

Pattern 1

Pattern 2

Pattern 3

Pattern 4

. . . . .

Fig.3

**INTERNATIONAL SEARCH REPORT**

| International application No. |
| --- |
| PCT/JP2017/016917 |

### A. CLASSIFICATION OF SUBJECT MATTER

C07K14/435(2006.01)i, C12N1/15(2006.01)i, C12N1/19(2006.01)i, C12N1/21
(2006.01)i, C12N5/10(2006.01)i, C12N15/09(2006.01)i, D01F4/02(2006.01)i

According to International Patent Classification (IPC) or to both national classification and IPC

### B. FIELDS SEARCHED

Minimum documentation searched (classification system followed by classification symbols)
C07K14/435, C12N1/15, C12N1/19, C12N1/21, C12N5/10, C12N15/09, D01F4/02

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN),
UniProt/GeneSeq

### C. DOCUMENTS CONSIDERED TO BE RELEVANT

| Category* | Citation of document, with indication, where appropriate, of the relevant passages | Relevant to claim No. |
| --- | --- | --- |
| X/A | WO 2011/113592 A1  (AMSILK GMBH),<br>22 September 2011 (22.09.2011),<br>claims; page 6, 7th paragraph; page 7, 5th paragraph; page 10, 2nd paragraph; page 10, 3rd paragraph; page 13, 1st paragraph; page 17, 2nd paragraph; page 20, 4th paragraph to page 21, 1st paragraph<br>& US 2013/0172478 A1    & EP 2547810 A1 | 1-14,16,17,<br>19-35/15,18 |
| A | WO 03/057727 A1  (NEXIA BIOTECHNOLOGIES, INC.),<br>17 July 2003 (17.07.2003),<br>claims<br>& US 2007/0260039 A1 | 1-35 |

☐  Further documents are listed in the continuation of Box C.          ☐  See patent family annex.

| | |
| --- | --- |
| *      Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    19 July 2017 (19.07.17) | Date of mailing of the international search report<br>    01 August 2017 (01.08.17) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

## REFERENCES CITED IN THE DESCRIPTION

### Patent documents cited in the description

- JP 2012055269 A
- JP 2005502347 A
- JP 2009505668 A
- JP 2014502140 A
- WO 2015042164 A
- JP S63248394 B
- JP S58110600 B
- JP S60221091 B
- US 4686191 A
- US 4939094 A
- US 5160735 A
- JP H231682 B
- JP H4278091 B
- JP H7170984 B
- JP H6133782 B
- JP 2002238569 A
- JP H2227075 B
- JP H5336963 B
- WO 9423021 A
- JP 5678283 B
- JP 4638735 B

### Non-patent literature cited in the description

- **ASAKURA et al.** Encyclopedia of Agricultural Science. Academic Press, 1994, vol. 4, 1-11
- *Microbial Cell Factories,* 2004
- *Science,* 2002, vol. 295, 472-476
- *Nucleic Acid Res.,* 1982, vol. 10, 6487
- *Methods in Enzymology,* 1983, vol. 100, 448
- *Proc. Natl. Acad. Sci. USA,* 1972, vol. 69, 2110
- *Gene,* 1982, vol. 17, 107
- *Molecular & General Genetics,* 1979, vol. 168, 111
- **TAKAHASHI et al.** *J. Bacteriol.,* 1983, vol. 156, 1130-1134
- **TAKAGI et al.** *Agric. Biol. Chem.,* 1989, vol. 53, 3099-3100
- **OKAMOTO et al.** *Biosci. Biotechnol. Biochem.,* 1997, vol. 61, 202-203
- *Agric. Biol. Chem.,* 1984, vol. 48, 669
- *Agric. Biol. Chem.,* 1989, vol. 53, 277
- *Proc. Natl. Acad. Sci. USA,* 1985, vol. 82, 4306
- *J. Bacteriol.,* 1990, vol. 172, 2392
- *J. Bacteriol.,* 1987, 1239-1245
- **UDAKA SHIGEZOU.** *Journal of the Agricultural Chemical Society of Japan,* 1987, vol. 61, 669-676
- *Appl. Microbiol. Biotechnol.,* 1989, vol. 30, 75-80
- *J. Biochem.,* 1992, vol. 112, 488-491
- *Appl. Environ. Microbiol.,* 1992, vol. 58, 525-531
- *J. Bacteriol.,* 1995, vol. 177, 745-749
- *Appl. Microbiol. Biotechnol.,* 1994, vol. 42, 358-363
- **MYERS, A. M. et al.** *Gene,* 1986, vol. 45, 299-310
- *Methods Enzymol.,* 1990, vol. 194, 182
- *Proc. Natl. Acad. Sci., USA,* 1984, vol. 81, 4889
- *J. Bacteriol.,* 1983, vol. 153, 163
- *Proc. Natl. Acad. Sci. USA,* 1978, vol. 75, 1929
- **COHEN et al.** calcium chloride method. *Proc. Natl. Acad. Sci. USA,* 1972, vol. 69, 2110
- *Mol. Gen. Genet.,* 1979, vol. 168, 111
- *J. Mol. Biol.,* 1971, vol. 56, 209
- Baculovirus Expression Vectors, A Laboratory Manual. W. H. Freeman and Company, 1992
- Current Protocols in Molecular Biology, Baculovirus Expression Vectors, A Laboratory Manual. W. H. Freeman and Company, 1992
- *Bio/Technology,* 1988, vol. 6, 47
- *Proc. Natl. Acad. Sci. USA,* 1987, vol. 84, 7413
- *Biosci. Biotechnol. Biochem.,* 1995, vol. 59, 1795-1797
- *Enzyme Microbiol Technol,* 1984, vol. 6, 386-389
- *Gene,* 1989, vol. 84, 329-334
- *BiosciBiotechnol Biochem,* 2000, vol. 64, 1416-1421
- *BiochemBiophys Res Commun,* 1983, vol. 112, 284-289
- *Gene,* 1983, vol. 26, 205-221
- *Mol Gen Genet,* 1999, vol. 261, 290-296
- *Proc Natl Acad Sci USA,* 1986, vol. 83, 4869-4873
- *Gene,* 1987, vol. 57, 21-26
- Molecular Cloning
- *Nature,* 1962, vol. 195, 788
- **PAULSON et al.** *J. Biol. Chem.,* 1989, vol. 264, 17619

- **LOWE et al.** *Proc. Natl. Acad. Sci. USA,* 1989, vol. 86, 8227

- *Genes Develop.,* 1990, vol. 4, 1288